(19) Europäisches Patentamt
European Patent Office
Office européen des brevets

(11) **EP 3 484 610 B1**

(12) **FASCICULE DE BREVET EUROPEEN**

(45) Date de publication et mention de la délivrance du brevet:
**26.08.2020 Bulletin 2020/35**

(21) Numéro de dépôt: **17748832.7**

(22) Date de dépôt: **12.07.2017**

(51) Int Cl.:
*B01J 20/06* (2006.01)       *B01J 20/28* (2006.01)
*A01N 25/04* (2006.01)       *A01N 59/00* (2006.01)
*A62D 3/00* (2006.01)        *A62D 3/176* (2007.01)
*A62D 3/30* (2007.01)        *A62D 3/33* (2007.01)
*A62D 101/02* (2007.01)      *A62D 101/24* (2007.01)
*A62D 101/43* (2007.01)      *G21F 9/00* (2006.01)
*G21F 9/28* (2006.01)

(86) Numéro de dépôt international:
**PCT/FR2017/051922**

(87) Numéro de publication internationale:
**WO 2018/011525 (18.01.2018 Gazette 2018/03)**

(54) **GEL DE DÉCONTAMINATION ADSORBANT ET PHOTOCATALYTIQUE ET PROCÉDÉ DE DÉCONTAMINATION DE SURFACES UTILISANT CE GEL.**

ADSORPTIONSMITTEL UND PHOTOKATALYTISCHES DEKONTAMINATIONSGEL SOWIE VERFAHREN ZUR DEKONTAMINATION VON OBERFLÄCHEN MIT DIESEM GEL

ADSORBENT AND PHOTOCATALYTIC DECONTAMINATION GEL, AND METHOD FOR DECONTAMINATING SURFACES USING SAID GEL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.07.2016 FR 1656758**

(43) Date de publication de la demande:
**22.05.2019 Bulletin 2019/21**

(73) Titulaire: **Commissariat à l'énergie atomique et aux énergies alternatives**
**75015 Paris (FR)**

(72) Inventeurs:
• **GOSSARD, Alban**
  **84000 Avignon (FR)**
• **FRANCES, Fabien**
  **30340 Rousson (FR)**
• **LEPEYTRE, Célia**
  **30650 Rocheford du Gard (FR)**

(74) Mandataire: **Brevalex**
**56, Boulevard de l'Embouchure**
**B.P. 27519**
**31075 Toulouse Cedex 2 (FR)**

(56) Documents cités:
EP-A1- 1 174 392            WO-A1-96/23051
WO-A1-2014/154817          WO-A1-2014/154818
DE-A1-102004 033 620       DE-A1-102004 033 621
DE-A1-102007 014 875       DE-A1-102007 025 562
FR-A1- 2 789 591           JP-A- H11 101 894

• Tiele Caprioli Machado ET AL: "Reduction of hexavalent chromium: photocatalysis and photochemistry and their application in wastewater remediation", Water Science and Technology, vol. 70, no. 1, 1 July 2014 (2014-07-01), pages 55-61, XP055665844, GB ISSN: 0273-1223, DOI: 10.2166/wst.2014.193

**Description**

**DOMAINE TECHNIQUE**

La présente invention a pour objet un gel de décontamination adsorbant et photocatalytique utilisable pour la décontamination de surfaces.

La présente invention a trait, en outre, à un procédé de décontamination de surfaces utilisant ce gel.

Le procédé et le gel selon l'invention permettent la décontamination de toutes sortes de surfaces telles que les surfaces en matériaux métalliques, plastiques, minéraux comme les matériaux vitreux.

Le procédé et le gel selon l'invention s'appliquent notamment, entre autres, à la décontamination des surfaces de matériaux poreux tels que les matériaux cimentaires comme les mortiers et les bétons ; les briques ; les plâtres ; et la pierre naturelle.

Le procédé et le gel selon l'invention permettent également l'élimination de toutes sortes d'espèces contaminantes (ou contaminants) et notamment des espèces contaminantes ioniques, chimiques, biologiques, ou nucléaires, radioactives.

Lorsque, par exemple, les espèces contaminantes éliminées par le gel sont des espèces contaminantes ioniques, alors le gel est dénommé gel de décontamination ionique.

Le domaine technique de l'invention peut ainsi, de manière générale, être défini comme étant celui de la décontamination de surfaces en vue d'éliminer les polluants, contaminants qui se trouvent sur cette surface et éventuellement sous cette surface, et dont la présence sur et sous ces surfaces n'est pas souhaitée.

**ÉTAT DE LA TECHNIQUE ANTÉRIEURE**

Les gels de décontamination ont été notamment développés pour réaliser la décontamination radiologique de surface, en particulier lors du démantèlement d'installations nucléaires.

Ainsi, dans le cadre de la décontamination nucléaire, des formulations gélifiées qui permettent de s'affranchir des problèmes liés au caractère pulvérulent du déchet sec, et d'accroître l'efficacité des procédés mettant en œuvre un gel ont fait l'objet des documents [1] et [2].

Ces documents [1] et [2] décrivent des gels colloïdaux inorganiques dits « gels aspirables », spécifiquement formulés pour être pulvérisés, puis pour sécher en se fracturant, tout en piégeant et confinant la contamination radioactive sous forme de paillettes aspirables et stockables.

Ces gels colloïdaux comprennent un agent viscosant inorganique à base de silice ou d'alumine, un ou plusieurs agents actifs de décontamination, et éventuellement un ou plusieurs tensio-actif(s).

Plus précisément, le document [1] décrit un gel constitué d'une solution colloïdale comprenant un agent viscosant inorganique, généralement de la silice ou de l'alumine, un agent actif de traitement qui est par exemple un acide ou une base inorganique telle que la soude ou la potasse, et éventuellement un agent oxydant ayant un potentiel normal d'oxydoréduction $E_0$ supérieur à 1,4 V en milieu acide fort tel que Ce(IV), Co(III), ou Ag(II).

Le document [2] décrit un gel constitué d'une solution colloïdale comprenant un agent viscosant organique, généralement de la silice ou de l'alumine, un tensio-actif, un acide ou une base inorganique, et éventuellement un agent oxydant ayant un potentiel normal d'oxydoréduction $E_0$ supérieur à 1,4 V en milieu acide fort tel que Ce(IV), Co(III), ou Ag(II).

Ces gels colloïdaux inorganiques, du fait des différents constituants entrant dans leur composition, ont une rhéologie qui permet leur pulvérisation sur une surface contaminée, puis leur adhésion à cette surface, même verticale, sans couler.

Cela permet ainsi un contact prolongé entre le contaminant et l'agent actif de décontamination, sans que les propriétés mécaniques du substrat ne soient altérées.

Suite à sa pulvérisation, le gel sèche, se fracture, et produit des résidus secs, appelés « paillettes », adhérant au substrat et qui sont par la suite évacués par brossage et/ou aspiration pour être directement conditionnés.

Les procédés de décontamination qui mettent en œuvre ces gels aspirables sont donc des procédés de décontamination par voie sèche, ne générant aucun effluent liquide et peu de résidus solides secs. En effet, ces résidus solides secs ne représentent en moyenne qu'un quart de la masse de gel initialement pulvérisée. De plus, ces procédés limitent le temps d'exposition des opérateurs à la contamination radioactive, du fait de leur mise en œuvre facile par pulvérisation puis aspiration des résidus secs, et du fait que la présence de l'opérateur n'est pas requise pendant le séchage du gel.

Les gels décrits dans les documents [1] et [2] peuvent être notamment qualifiés de gels de décontamination « ioniques » car la pollution qu'ils ont pour but d'éliminer se présente notamment sous la forme d'ions.

Ces gels doivent leur efficacité à une attaque chimique de la surface à décontaminer grâce à la présence d'un acide, ou à la présence d'un agent organique puissant comme l'oxyde de cérium.

Or, dans certains cas, il est nécessaire de préserver la surface à décontaminer, et d'éviter sa corrosion, tout en assurant une extraction, élimination, des polluants, contaminants présents sur cette surface.

De plus, après séchage et aspiration de ces gels, les paillettes de gel peuvent relarguer les espèces polluantes, contaminantes, piégées, captées par les gels, lorsque les paillettes de gel sec entrent en contact avec de l'eau.

En effet, les espèces polluantes sont simplement « encapsulées » dans les paillettes de gel sec qui ne possèdent donc pas de pouvoir « confinant ».

Les gels de décontamination tels que ceux décrits dans les documents [1] et [2] présentent donc l'inconvénient de causer une corrosion, détérioration de la surface à décontaminer et de former des paillettes qui ne confinent pas les espèces contaminantes, polluantes, et qui peuvent relarguer ces espèces polluantes notamment dans le cas où les paillettes de gel sec entrent en contact avec un liquide tel que de l'eau.

Il existe donc, au regard de ce qui précède, un besoin pour un gel de décontamination « ionique » qui permette un transfert efficace des espèces contaminantes ioniques, sans corroder, attaquer, détériorer cette surface.

Il existe en outre un besoin pour un tel gel de décontamination « ionique » qui assure un confinement des espèces contaminantes, polluantes, au sein du gel sec, après le traitement des surfaces contaminées et qui évite le relargage de ces espèces polluantes, contaminantes, notamment dans le cas où les paillettes de gel sec entrent en contact avec un liquide tel que de l'eau.

D'autres gels ont été développés pour réaliser la décontamination biologique ou chimique de surfaces.

De tels gels de décontamination biologique font l'objet des documents [3] et [4].

Ces gels comprennent en tant qu'agents actifs de décontamination biologique des agents oxydants puissants et plus particulièrement de l'hypochlorite de sodium.

Plus précisément, le document [3] décrit un gel de décontamination biologique constitué par une solution colloïdale comprenant au moins un agent viscosant inorganique, généralement de la silice ou de l'alumine, au moins un agent actif de décontamination biologique, au moins un polymère super absorbant, au moins un agent tensio-actif, et le reste de solvant.

Le document [4] décrit un gel de décontamination biologique constitué par une solution colloïdale comprenant au moins un agent viscosant inorganique, généralement de la silice ou de l'alumine, un agent actif de décontamination biologique constitué par la combinaison d'une base minérale choisie parmi les hydroxydes de métaux alcalins, les hydroxydes de métaux alcalinoterreux, et leurs mélanges, et d'un agent oxydant stable en milieu basique choisi parmi les permanganates, les persulfates, l'ozone, les hypochlorites et leurs mélange, éventuellement au moins un agent tensio-actif, et le reste de solvant, et le gel ne contenant pas de polymère super-absorbant.

Dans les documents [1], [2], [3] et [4], le $TiO_2$ n'est pas mentionné parmi les agents viscosants inorganiques.

Les gels des documents [3] et [4], qui sont des gels pulvérisables et aspirables, peuvent attaquer la surface de certains métaux, comme l'aluminium, notamment à cause de la présence de chlore dans leur formulation. Le document [5] divulgue un produit de nettoyage de surfaces solides pouvant être sous forme de gel comprenant un matériau photocatalytique à base de $TiO_2$ éventuellement modifié, un agent hydratant, un agent tensio-actif, un solvant organique et de l'eau .

Ces gels ont de bonnes performances en ce qui concerne l'élimination des espèces toxiques biologiques, mais ils possèdent une stabilité chimique limitée. Par conséquent, la durée pendant laquelle ils peuvent être conservés, stockés à la température ambiante, tout en gardant intacte leur efficacité de décontamination, est faible.

En effet, la quantité de chlore actif contenue dans l'hypochlorite de sodium diminue significativement au bout de 6 mois si le gel n'est pas conservé au frais.

Il existe donc un besoin pour un gel de décontamination biologique et chimique qui soit efficace sur une longue durée, c'est-à-dire qui puisse être stocké pendant une longue durée à température ambiante, tout en gardant intacte son efficacité de décontamination lorsqu'il est utilisé.

Il existe aussi un besoin pour un tel gel de décontamination biologique et chimique qui assure une décontamination efficace d'une surface et qui préserve la surface traitée, c'est-à-dire qui ne corrode pas, n'attaque pas, et ne détériore pas cette surface, autrement dit qui ne produise aucune altération, chimique, mécanique ou physique de la surface traitée.

Le but de la présente invention est de fournir un gel de décontamination qui réponde entre autres aux besoins et exigences énumérés plus haut.

Le but de la présente invention est encore de fournir un gel de décontamination qui ne présente pas les inconvénients défauts, limitations et désavantages des gels de décontamination de l'art antérieur et qui résolve les problèmes des gels de décontamination de l'art antérieur, notamment des gels faisant l'objet des documents [1], [2], [3], et [4].

## EXPOSÉ DE L'INVENTION

Ce but, et d'autres encore, sont atteints, conformément à l'invention, par un gel de décontamination adsorbant et photocatalytique, constitué par une solution colloïdale comprenant, de préférence constituée par :

- 15% à 20% en masse, mieux 15% à 20% en masse la valeur 15% étant exclue, mieux encore 16% à 20% en masse, par exemple 20% en masse de $TiO_2$, éventuellement dopé, par rapport à la masse du gel;
- éventuellement, 0,01% à 10% en masse, de préférence 0,1% à 5% en masse, par rapport à la masse du gel, d'au moins un colorant et/ou d'au moins un pigment;
- éventuellement, 0,1% à 2% en masse par rapport à la masse du gel, d'au moins un agent tensio-actif;
- éventuellement, 0,05% à 5% en masse, de préférence 0,05% à 2% en masse par rapport à la masse du gel, d'au moins un polymère super-absorbant ;
- et le reste de solvant, ledit solvant étant choisi parmi les mélanges d'eau dans une proportion de 40% à 56 % en masse, par exemple 40%, 42,5%, ou 56% en masse, et d'éthanol, dans une proportion de 24% à 42,5 % en masse, mieux de 24% à 40% en masse, par exemple 24 %, 40 %, ou 42,5 % en masse par rapport à la masse du gel. et ledit gel ayant un pH supérieur ou égal à 4.

Par « reste de solvant », on entend que le solvant est toujours présent dans la solution colloïdale et que la quantité de solvant est une quantité telle que, lorsqu'elle est ajoutée aux quantités des composants de la solution colloïdale autres que le solvant (que ces composants soient des composants obligatoires ou éventuels cités plus haut, ou encore d'autres composants additionnels optionnels cités ou non cités), la quantité totale de tous les composants de la solution colloïdale est de 100% en masse.

Dans tous les cas, il est bien évident, que la quantité totale de tous les composants de la solution colloïdale (que ces composants soient des composants obligatoires ou éventuels cités plus haut, ou encore d'autres composants additionnels optionnels cités ou non cités) est bien entendu de 100% en masse. Le gel selon l'invention est une solution colloïdale, ce qui signifie que le gel selon l'invention contient des particules solides inorganiques, minérales, de $TiO_2$, en tant qu'agent viscosant dont les particules élémentaires, primaires, ont une taille moyenne généralement de 2 à 200 nm.

Du fait de la mise en œuvre d'un agent viscosant généralement exclusivement inorganique, sans agent viscosant organique, la teneur en matières organiques du déchet solide final obtenu à l'issue de la mise en œuvre du gel selon l'invention est généralement inférieure à 4% en masse, de préférence inférieure à 2% en masse, ce qui constitue encore un autre avantage des gels selon l'invention.

Ces particules solides, minérales, inorganiques jouent le rôle de viscosant pour permettre à la solution, par exemple la solution aqueuse, de se gélifier et ainsi d'adhérer aux surfaces de la pièce à traiter, décontaminer, quelle que soit leur géométrie, leur forme, leur taille, et où que se trouvent les contaminants à éliminer.

Le gel de décontamination selon l'invention peut être appelé gel de décontamination colloïdal à base de $TiO_2$.

Le gel selon l'invention permet la décontamination de surfaces contaminées, polluées par toutes sortes d'espèces polluantes, contaminantes, par exemple d'espèces contaminantes, ioniques, chimiques ou biologiques.

Le gel selon l'invention se différencie fondamentalement des gels de l'art antérieur, tels que ceux des documents [1], [2], [3], et [4], en ce qu'il contient du $TiO_2$ en tant qu'agent viscosant inorganique et non de la silice ou de l'alumine.

Autrement dit dans le gel de décontamination selon l'invention, les particules colloïdales habituellement utilisées dans les gels de décontamination de l'art antérieur, telles que les particules colloïdales de silice ou d'alumine sont remplacées par des particules colloïdales de $TiO_2$.

En outre, le gel selon l'invention contient une quantité extrêmement spécifique de $TiO_2$, à savoir une teneur de 15% à 20% en masse, mieux de 15% à 20% en masse la valeur 15% étant exclue, mieux encore de 16% à 20% en masse, par exemple de 20% en masse de $TiO_2$, éventuellement dopé, par rapport à la masse du gel.

Il a été constaté, selon l'invention qu'un gel présentant une teneur, concentration en $TiO_2$ se situant dans la plage spécifique mentionnée plus haut, et dans la plage de 15% à 20% en masse, mieux dans la plage de 15% à 20% en masse la valeur 15% étant exclue, et avec un pH supérieur ou égal à 4, possédait de manière surprenante une excellente tenue sur des parois non-horizontales telles que des plafonds ou des murs.

Il est à noter que le $TiO_2$ est stable sur de longues durées, pouvant atteindre par exemple au moins 1 an.

L'incorporation de $TiO_2$ dans un gel de décontamination, à la teneur très spécifique mentionnée plus haut, c'est à dire dans la plage de 15% à 20% en masse, mieux dans la plage de 15% à 20% en masse la valeur 15% étant exclue de $TiO_2$ par rapport à la masse du gel, n'a jamais été décrite ni suggérée dans l'art antérieur et notamment dans les documents [1], [2], [3], et [4], cités plus haut.

Le gel selon l'invention, essentiellement du fait qu'il comprend du $TiO_2$ en tant qu'agent viscosant inorganique, à la place de la silice ou de l'alumine, répond à l'ensemble des besoins et exigences mentionnés plus haut. Il ne présente pas les inconvénients, défauts, limitations et désavantages des gels de l'art antérieur tels que ceux tels que les gels des documents [1], [2], [3], et [4] qui contiennent de la silice ou de l'alumine, et non du $TiO_2$, en tant qu'agent viscosant.

Le gel selon l'invention permet d'atteindre les buts mentionnés plus haut, et résout les problèmes présentés par les gels de décontamination de l'art antérieur tels que ceux décrits dans les documents [1], [2], [3], et [4] sans en présenter les inconvénients, notamment en termes de corrosion de la surface, de relargage des espèces contaminantes, de faible stabilité et de durée de conservation limitée, et tout en possédant une efficacité de décontamination élevée quelle que soit l'espèce contaminante, polluante.

De manière surprenante, dans le gel selon l'invention, le dioxyde de titane joue donc non seulement le rôle d'agent viscosant inorganique mais aussi le rôle d'agent de décontamination, mais avec l'avantage par rapport aux agents actifs de décontamination habituellement mis en œuvre dans les gels de décontamination de l'art antérieur, tels que ceux des documents [1], [2], [3], et [4] de présenter une innocuité totale vis-à-vis de ces surfaces et d'être, comme on l'a déjà mentionné plus haut, stable sur de longues durées.

Le gel selon l'invention possède ainsi une durée de conservation d'au moins un an, de préférence d'au moins deux ans, par exemple de un à deux ans, c'est-à-dire que le gel selon l'invention est stable pendant cette durée, qu'il ne subit aucune dégradation de quelque nature que ce soit pendant cette durée.

Du fait que, dans le gel selon l'invention, le $TiO_2$ joue le rôle d'agent actif de décontamination, le gel selon l'invention gel ne comporte pas d'agents actifs de décontamination agressifs, corrosifs, susceptibles de provoquer une détérioration, un endommagement, une attaque des surfaces à traiter pour les surfaces à traiter tels que les agents actifs de décontamination mentionnés dans les documents [1], [2], [3], et [4].

Le gel selon l'invention, mais aussi le procédé de décontamination qui met en œuvre ce gel (décrit plus bas) mettent à profit le pouvoir adsorbant et le pouvoir photocatalytique du dioxyde de titane sous rayonnement lumineux pour éliminer avec une très grande efficacité les espèces polluantes, contaminantes, de la surface d'un substrat solide contaminé, pollué, par ces espèces contaminantes quelles qu'elles soient : ioniques, chimiques, biologiques, nucléaires ou radioactives. Une description détaillée des espèces contaminantes qui peuvent être éliminées par le procédé selon l'invention est donnée plus bas dans le cadre de la description du procédé.

Autrement dit, le gel selon l'invention possède un pouvoir adsorbant, des propriétés adsorbantes, dues au $TiO_2$, notamment dans le cas d'espèces contaminantes ioniques, et d'espèces contaminantes qui sont des composés chimiques organiques, ainsi que des propriétés « photocatalytiques » qui permettent par exemple un changement de degré d'oxydation des composés ioniques ou la réduction de composés organiques.

Comme on l'a déjà indiqué plus haut, $TiO_2$ est stable sur de longues durées, pouvant atteindre au moins 1 an.

Les propriétés photocatalytiques et adsorbantes qui sont conférées au gel par la présence de $TiO_2$, et qui permettent une réduction de la toxicité des espèces contaminantes, sont ainsi conservées sur de longues durées, pouvant atteindre au moins 1 an, contrairement aux gels de l'art antérieur.

En d'autres termes, un des effets remarquables et surprenants des gels selon l'invention est que, comme les propriétés adsorbantes et photoactives du gel proviennent du $TiO_2$, ces propriétés sont conservées sur de longues durées, alors que dans le cas de gels, notamment à l'alumine où à la silice, incorporant des solutions décontaminantes en tant qu'agents actifs de décontamination, le vieillissement peut entraîner une perte de l'efficacité de ces solutions décontaminantes.

Le gel selon l'invention combine ce pouvoir adsorbant et ce pouvoir photocatalytique du dioxyde de titane, avec des propriétés rhéologiques adéquates qui permettent entre autres d'assurer l'application du gel par pulvérisation, sa tenue sur une paroi verticale ou un plafond, et de rendre le gel sec, qui se présente généralement sous la forme de paillettes, facilement éliminable par brossage ou par aspiration et de préférence par aspiration. Le gel selon l'invention peut ainsi être qualifié de gel « pulvérisable » et de « gel aspirable ».

On peut dire que la présence de dioxyde de titane en tant qu'agent viscosant, gélifiant, inorganique dans le gel selon l'invention, permet que le gel selon l'invention soit pulvérisable, permet que le gel selon l'invention soit aspirable après application sur la surface à dépolluer, décontaminer, et séchage, et enfin, permet que le gel possède une longue durée de conservation.

Les termes « gel aspirable » sont largement utilisés dans ce domaine de la technique et ont une définition bien connue qui a été rappelée plus haut.

Les propriétés rhéologiques du gel selon l'invention sont dues aux propriétés viscosantes, rhéofluidifiantes des particules de dioxyde de titane dispersées au sein du solvant du gel, ainsi qu'au pH du gel, supérieur ou égal à 4, qui permet d'ajuster la viscosité du gel, et d'assurer notamment l'application du gel par pulvérisation et sa tenue, son maintien sur une paroi vertical ou un plafond.

Il n'avait pas été mis en évidence jusqu'alors que le $TiO_2$ était un agent viscosant aux propriétés rhéofluidifiantes tout comme la silice et l'alumine.

Les gels selon l'invention qui contiennent en tant qu'agent viscosant le $TiO_2$ possèdent de ce fait, de manière étonnante, les propriétés d'un fluide rhéofluidifiant, thixotrope, leur viscosité pouvant varier en fonction du cisaillement et au cours du temps. En outre, selon l'invention, il a été mis en évidence qu'à partir d'une concentration suffisamment importante en $TiO_2$, à savoir 15% en masse, mieux plus de 15% en masse (la valeur 15% est alors exclue), mieux encore 16% en masse, et avec une formulation adaptée, c'est-à-dire avec un pH supérieur ou égal à 4, on peut obtenir une suspension assez stable et visqueuse pour pouvoir être appelée « gel ».

De plus, le $TiO_2$, que contient le gel selon l'invention, en tant que viscosant inorganique, minéral, influence de manière inattendue le séchage du gel selon l'invention et la granulométrie du résidu obtenu.

En effet, le gel sec se présente sous la forme de particules de taille contrôlée, plus précisément de paillettes solides millimétriques, dont la taille moyenne va généralement de 1 à 10 mm, de préférence de 2 à 5 mm, grâce notamment au fait que l'agent viscosant inorganique est constitué par du $TiO_2$.

Précisons que la taille des particules, telles que des paillettes, correspond généralement à leur plus grande dimension.

En d'autres termes, les particules solides minérales de $TiO_2$ du gel selon l'invention, outre leur rôle de viscosant, jouent également un rôle fondamental lors du séchage du gel car elles assurent la fracturation du gel pour aboutir à un déchet sec sous forme de paillettes.

Plus exactement, lors de la préparation du gel, les particules de $TiO_2$ sont dans un premier temps dispersées au sein du solvant, qui est selon l'invention un solvant hydro-alcoolique, et le pH est augmenté jusqu'à une valeur supérieure ou égale à 4, afin d'ajuster la viscosité du système permettant ainsi sa pulvérisation puis sa tenue sur la surface à décontaminer, notamment sur une paroi verticale ou un plafond.

Le pH du gel peut être aussi ajusté afin qu'il n'y ait pas ou peu d'attaque chimique de la surface au cours du traitement c'est-à-dire que le pH est alors ajusté pour que le gel soit légèrement acide, ou légèrement basique, voire neutre.

En effet, un des avantages du gel selon l'invention est que l'on peut facilement en faire varier le pH.

Ainsi, si l'on ne veut pas attaquer la surface, on peut adapter le pH à la surface à décontaminer.

Par contre, si l'on souhaite une légère attaque de la surface, le pH du gel peut être modifié pour permettre cette attaque.

Ou bien, le pH du gel peut être ajusté afin que le gel soit fortement basique, par exemple supérieur ou égal à 9, et pour conférer un pouvoir dégraissant au gel.

Quel que soit le pH du gel selon l'invention, la surface traitée par le gel selon l'invention, est décontaminée, et peut être éventuellement dégraissée, et dans tous les cas elle demeure intacte, elle n'est pas attaquée, corrodée. Cette surface est donc éventuellement réutilisable après décontamination.

Selon l'invention, il est donc possible de formuler un gel neutre, basique ou faiblement acide qui combine le pouvoir adsorbant et le pouvoir photocatalytique du dioxyde de titane sous rayonnement lumineux, avec des propriétés rhéologiques permettant notamment que le gel sec soit pulvérisable et aspirable.

En effet, le dioxyde de titane possède d'excellentes propriétés adsorbantes des ions et des composés chimiques organiques.

Plus précisément, la sorption d'eau à la surface du dioxyde de titane entraîne la formation de groupements hydroxyles OH qui sont des sites préférentiels pour la sorption d'ions par des mécanismes de complexation de surface.

Le dioxyde de titane est ainsi capable de capter à sa surface un grand nombre d'ions.

Le gel selon l'invention, à base de $TiO_2$, utilise cette propriété d'adsorption des ions, cette propriété adsorbante des ions, et donc d'extraction des ions que possède le $TiO_2$ pour améliorer le captage des espèces contaminantes ioniques présentes sur une surface à décontaminer, mais sans attaque chimique de la surface.

De la même manière, le gel selon l'invention, à base de $TiO_2$, utilise cette propriété d'adsorption des composés chimiques organiques, cette propriété adsorbante des composés chimiques organiques, et donc d'extraction des composés chimiques organiques que possède le $TiO_2$ pour améliorer le captage des espèces contaminantes qui sont des composés chimiques organiques présents sur une surface à décontaminer, mais sans attaque chimique de la surface.

De plus, après traitement, les espèces contaminantes, polluantes contenues dans les paillettes de gels restent liées chimiquement au $TiO_2$ assurant ainsi un confinement de cette pollution et minimisant ainsi le risque de dissémination de la pollution dans l'environnement en cas de lixiviation accidentelle des paillettes de gel sec.

En d'autres termes grâce au pouvoir d'adsorption que possède le $TiO_2$, qui permet d'adsorber les espèces contaminantes, polluantes, notamment ioniques, à sa surface, l'espèce contaminante, notamment ionique, extraite est fixée chimiquement dans les paillettes limitant alors un éventuel relargage dans l'eau dans le cas où celle-ci viendrait à lixivier les paillettes de gel sec au cours du stockage du gel.

En outre, le dioxyde de titane possède un pouvoir photocatalytique très fort.

Le dioxyde titane est en effet fortement actif et permet la réduction photochimique de nombreuses espèces contaminantes, polluantes, et notamment du chrome hexavalent en chrome trivalent.

Dans le cas d'une pollution, contamination, par une espèce contaminante qui est un élément multivalent sous forme ionique, comme le chrome Cr, ou un métal lourd comme l'arsenic As ou le mercure Hg, un changement de degré d'oxydation de l'espèce contaminante peut entraîner une diminution drastique de sa toxicité.

Le taux de réduction du chrome hexavalent est notamment fortement augmenté pour des gels acides (généralement de pH<6 : le gel a donc alors un pH de 4 à 6) et sous l'action d'un rayonnement UV.

En résumé la propriété photo-catalytique du gel due au $TiO_2$, permet la réduction du degré d'oxydation de polluants ioniques engendrant alors une baisse de leur toxicité (cas du Cr(VI) dans les différents exemples).

Ainsi, le gel selon l'invention répond à un très gros besoin existant auquel ne répondaient pas les gels de l'art antérieur.

Il a également été montré (voir plus bas et exemples) que l'ajout d'éthanol permet d'améliorer l'efficacité réductrice du gel selon l'invention, par exemple le taux de réduction du chrome hexavalent, en agissant au sein du système en tant qu'inhibiteur de recombinaison des paires électrons-trous, rendant ainsi plus facile la capture d'un électron par le chrome (VI) et donc sa réduction.

Le caractère photocatalytique du $TiO_2$ permet également la destruction des polluants chimiques et biologiques.

En effet, sous l'action d'un rayonnement UV, des radicaux hydroxyles se forment par oxydation de l'eau adsorbée en surface du $TiO_2$.

Ces radicaux réagissent avec les polluants chimiques ou biologiques pour les dégrader et ainsi annihiler leur toxicité.

On peut dire que le gel selon l'invention possède donc, grâce à un phénomène mixte d'adsorption du contaminant au sein de la phase gel, et de pouvoir photocatalytique du $TiO_2$, un triple pouvoir, une triple action, à savoir :

- un pouvoir, une action d'élimination des espèces contaminantes, polluantes ;
- un pouvoir de fixation des espèces contaminantes, polluantes, au sein des paillettes de gel sec ;
- un pouvoir de réduction de la toxicité des espèces contaminantes, polluantes, et ce quelles que soient ces espèces polluantes, contaminantes, qu'il s'agisse par exemple d'espèces polluantes, contaminantes ioniques, chimiques ou biologiques.

On note de plus que, du fait de la stabilité chimique du $TiO_2$ dans le solvant spécifique du gel selon l'invention, qui est un solvant hydro-alcoolique, le gel selon l'invention peut être conservé, stocké pendant une très longue durée pouvant atteindre par exemple jusqu'à 1 an et même 2 ans, voire plus.

Pour conclure, le gel selon l'invention permet la décontamination de manière très simple de surfaces polluées par des espèces contaminantes ioniques, mais également par des espèces contaminantes chimiques, biologiques, radioactives ou nucléaires grâce à la combinaison de l'efficacité d'adsorption et des propriétés photocatalytiques du dioxyde de titane sous rayonnement lumineux.

En outre, le gel selon l'invention possède les principaux avantages connus des gels de décontamination, à savoir : le faible coût, la facilité de mise en œuvre notamment par pulvérisation, et la facilité de gestion des déchets finaux qui se présentent généralement sous la forme de paillettes aspirables, sans production d'effluents liquides.

De plus, la formulation du gel ne comporte pas d'agent actif de décontamination agressif pour les surfaces à traiter.

Une autre caractéristique fondamentale du gel selon l'invention, est qu'il contient un solvant très spécifique choisi parmi les mélanges d'eau dans une proportion de 40% à 56 % en masse, par exemple 40%, 42,5%, ou 56% en masse, et d'éthanol, dans une proportion de 24% à 42,5 % en masse, de préférence 24% à 40% en masse, par exemple 24 %, 40 %, ou 42,5 % en masse par rapport à la masse du gel.

Comme on l'a déjà indiqué plus haut, il a été montré (voir exemples) que l'ajout d'éthanol dans le gel, permet d'améliorer l'efficacité réductrice du gel, par exemple le taux de réduction du chrome hexavalent, en agissant au sein du système en tant qu'inhibiteur de recombinaison des paires électrons-trous, rendant ainsi plus facile la capture d'un électron par le chrome (VI) et donc sa réduction.

De plus, il a été mis en évidence, que, de manière surprenante, la présence d'éthanol dans le gel selon l'invention (voir exemples) permettait d'augmenter le seuil d'écoulement du gel par rapport à un gel aqueux dont le solvant est constitué uniquement par de l'eau. Cela permet donc de déposer une épaisseur de gel plus importante sur des parois non-horizontales telles que des murs ou des plafonds.

Autrement dit, la présence d'éthanol dans le gel selon l'invention permet d'améliorer encore la tenue du gel sur des parois qui ne sont pas horizontales telles que des plafonds ou des murs (voir exemples) par rapport à un gel aqueux dont le solvant est constitué uniquement par de l'eau.

Comme on l'a déjà indiqué plus haut, le gel selon l'invention peut avoir un pH faiblement acide de 4 (4 inclus) à moins de 7 (c'est-à-dire de 4 à 7, 7 étant exclus); un pH neutre de 7 ; ou un pH faiblement basique de plus de 7 à moins de 9 ; ou encore un pH très basique de 9 ou plus.

Un pH préféré est de 5 (inclus) à 9 (inclus).

Des valeurs de pH particulièrement préférées sont 5, 5,5 et 9.

Quel que soit le pH du gel selon l'invention, aucune corrosion, attaque, détérioration, agression, de la surface traitée n'est généralement observée, et cette surface demeure intacte.

Avantageusement, le pH est ajusté par addition d'une base minérale.

Cette base minérale peut être choisie, par exemple, parmi la soude NaOH, la potasse KOH, et leurs mélanges.

Cette base minérale ne joue généralement pas le rôle d'agent de décontamination, comme dans les procédés de l'art antérieur.

Cependant elle peut seulement, éventuellement, donner aux gels des propriétés dégraissantes, lorsque le pH du gel est supérieur ou égal à 9, et si des contaminants sont présents dans la graisse, alors on pourrait éventuellement considérer que cette base joue bien le rôle d'agent de décontamination, le TiO$_2$ servant alors d'adsorbant des contaminants tels que des radionucléides présents dans la graisse.

La concentration de la base minérale dans le gel est la concentration nécessaire pour ajuster le pH du gel à la valeur souhaitée, supérieure (ou égale) à 4 et avantageusement entre 5 et 9.

Avantageusement, la concentration de la base minérale peut être de 1.10$^{-3}$ M à 2.10$^{-2}$ mol/L de gel.

Une telle concentration ne permet pas à cette base inorganique de jouer le rôle d'agent de contamination et ne conduit à aucune corrosion, détérioration, attaque de la surface à décontaminer.

Une telle concentration est faible et peut être généralement négligée par rapport à la masse de solvant et à la masse de TiO$_2$ dans le gel.

Le gel selon l'invention peut contenir en outre, éventuellement, au moins un colorant et/ou au moins un pigment.

Avantageusement le pigment est un pigment minéral. On pourra à cet égard se reporter au document WO-A1-2014/154817.

Il n'existe aucune limitation quant au pigment minéral qui est incorporé dans le gel de décontamination selon l'invention.

Généralement, le pigment minéral est choisi parmi les pigments minéraux qui sont stables dans le gel.

Par pigment stable, on entend généralement que le pigment ne présente pas de changement stable de sa couleur dans le temps, lors du stockage du gel pendant une durée minimale de 6 mois.

Il n'y a aucune limitation quant à la couleur de ce pigment, qui est généralement la couleur qu'il va communiquer au gel. Ce pigment peut être de couleur noire, rouge, bleue, verte, jaune, orange, violette, marron, etc., et même blanche.

Généralement, le gel a donc une couleur identique à la couleur du pigment qu'il contient. Il est toutefois possible que le gel possède une couleur qui diffère de la couleur du pigment qu'il contient mais cela n'est pas recherché.

Le pigment, notamment lorsqu'il est blanc, est généralement différent de l'agent viscosant inorganique.

Avantageusement, le pigment minéral est choisi de telle sorte qu'il donne au gel (c'est-à-dire au gel à l'état humide, avant séchage) une couleur différente de la couleur d'une surface à décontaminer sur laquelle le gel est appliqué.

Avantageusement, le pigment minéral est un pigment micronisé, et la taille moyenne des particules du pigment minéral peut être de 0,05 à 5 $\mu$m, de préférence de 0,1 à 1 $\mu$m.

Le fait que le pigment soit micronisé permet d'éviter qu'il ne modifie la rhéologie et l'aptitude à la pulvérisation du gel (« pulvérisabilité ») car le pigment a alors la même taille micrométrique qui est généralement celle de l'agent viscosant inorganique, tel que les agrégats d'alumine.

Avantageusement, le pigment minéral est choisi parmi les oxydes de métal (métaux) et/ou de métalloïde(s), les hydroxydes de métal (métaux) et/ou de métalloïde(s), les oxyhydroxydes de métal (métaux) et/ou de métalloïde(s), les ferrocyanures et ferricyanures de métal (métaux), les aluminates de métal (métaux), et leurs mélanges.

De préférence, le pigment minéral est choisi parmi les oxydes de fer, de préférence micronisés, et leurs mélanges.

Les oxydes de fer peuvent avoir différentes couleurs, ils peuvent être par exemple jaunes, rouges, violets, oranges, marrons, ou noirs.

En effet, les pigments d'oxyde de fer sont reconnus pour avoir un bon pouvoir couvrant et une grande résistance aux acides et aux bases.

Pour une incorporation dans un gel de décontamination, les oxydes de fer présentent les meilleures performances en termes de stabilité et de pouvoir colorant. Ainsi, une teneur en oxyde de fer de 0,1%, voire 0,01% en masse suffit à fortement colorer le gel sans en modifier les propriétés.

Comme on l'a déjà indiqué plus haut, le fait que le pigment d'oxyde de fer soit de préférence micronisé permet d'éviter qu'il ne modifie la rhéologie et l'aptitude à la pulvérisation du gel (« pulvérisabilité ») car le pigment a alors une taille micrométrique, à savoir une taille qui est généralement celle de l'agent viscosant inorganique, tel que les agrégats d'alumine.

Des oxydes de fers micronisés sont disponibles auprès de la société Rockwood® sous la dénomination commerciale Ferroxide®.

On peut citer entre autres le Ferroxide® 212 M qui est un oxyde de fer rouge micronisé ave une taille moyenne des particules de 0,1 $\mu$m et le Ferroxide® 228 M qui est un oxyde de fer rouge micronisé avec une taille moyenne des particules de 0,5 $\mu$m.

En plus et/ou à la place des oxydes de fer, d'autres oxydes ou hydroxydes de métaux ou de métalloïdes colorés peuvent être incorporés dans le gel selon l'invention, en fonction du pH du gel, on peut notamment citer l'oxyde de vanadium (V$_2$O$_5$) qui est orange, l'oxyde de manganèse (MnO$_2$) qui est noir, l'oxyde de cobalt qui est bleu ou vert, et les oxydes de terres rares. Cependant les oxydes de fer sont préférés pour les raisons précisées plus haut.

Parmi les oxyhydroxydes, on peut citer la goethite, c'est-à-dire l'oxyhydroxyde de fer FeOOH, qui est très colorée.

A titre d'exemple de ferrocyanure de métal, on peut citer, le bleu de Prusse, c'est-à-dire le ferrocyanure ferrique, et à titre d'exemple d'aluminate, on peut citer le bleu de cobalt, c'est-à-dire l'aluminate de cobalt.

L'incorporation dans le gel selon l'invention d'un pigment minéral permet de mieux visualiser le gel humide puis les résidus secs quel que soit le substrat sur lequel est appliqué le gel.

Le gel selon l'invention peut contenir en outre, éventuellement, au moins un polymère super-absorbant.

Par « polymère super-absorbant » également dénommé « PSA » ou « SAP », on entend généralement un polymère capable, à l'état sec, d'absorber spontanément au moins 10 fois, de préférence au moins 20 fois son poids de liquide aqueux, en particulier d'eau et notamment d'eau distillée.

Certains « SAP » peuvent absorber jusqu'à et même plus de 1000 fois leur poids de liquide.

De tels polymères super-absorbants sont notamment décrits dans l'ouvrage « Absorbent Polymer Technology, Studies in Polymer Science 8 » de L. BRANNON-PAPPAS et R. HARLAND, édition Elsevier, 1990, auquel on pourra se référer.

Par absorption spontanée, on entend un temps d'absorption allant jusqu'à environ une heure.

Le polymère super-absorbant peut avoir une capacité d'absorption d'eau allant de 10 à 2000 fois son propre poids, de préférence de 20 à 2000 fois son propre poids (soit 20 g à 2000 g d'eau absorbée par gramme de polymère absorbant), de préférence encore de 30 à 1500 fois, et en particulier de 50 à 1000 fois.

Ces caractéristiques d'absorption d'eau s'entendent dans les conditions normales de température (25°C) et de pression (760 mm Hg soit 100000 Pa) et pour de l'eau distillée.

Le PSA éventuellement contenu dans le gel de décontamination selon l'invention peut être choisi parmi les poly(méth)acrylates de sodium, les amidons greffés par un polymère (méth)acrylique, les amidons hydrolysés greffés par un polymère (méth)acrylique ; les polymères à base d'amidon, de gomme, et de dérivé cellulosique ; et leurs mélanges.

Plus précisément, le PSA que l'on peut éventuellement utiliser dans le gel selon l'invention peut être par exemple choisi parmi :

- les polymères résultant de la polymérisation avec réticulation partielle de monomères à insaturation éthylénique hydrosolubles, tels que les polymères acryliques, méthacryliques (issus notamment de la polymérisation de l'acide acrylique et/ou méthacrylique et/ou de monomères acrylate et/ou méthacrylate) ou vinyliques, en particulier les poly(méth)acrylates réticulés et neutralisés, notamment sous forme de gel ; et les sels notamment les sels alcalins tels que les sels de sodium ou de potassium de ces polymères ;
- les amidons greffés par des polyacrylates ;
- les copolymères acrylamide/acide acrylique, notamment sous forme de sels de sodium ou de potassium ;
- les amidons greffés acrylamide/acide acrylique, notamment sous forme de sels de sodium ou de potassium ;
- les sels de sodium ou de potassium de carboxyméthylcellulose ;
- les sels notamment les sels alcalins, d'acides polyaspartiques réticulés ;
- les sels notamment les sels alcalins, d'acides polyglutamiques réticulés.

En particulier, on peut utiliser comme « SAP » un composé choisi parmi :

- les polyacrylates de sodium ou de potassium réticulés vendus sous les dénominations SALSORB CL 10, SALSORB CL 20, FSA type 101, FSA type 102 (Allied Colloids) ; ARASORB S-310 (Arakawa Chemical) ; ASAP 2000, Aridall 1460 (Chemdal) ; KI-GEL 201-K (Siber Hegner) ; AQUALIC CA W3, AQUALIC CA W7, AQUALIC CA W10; (Nippon Shokuba) ; AQUA KEEP D 50, AQUA KEEP D 60, AQUA KEEP D 65, AQUA KEEP S 30, AQUA KEEP S 35, AQUA KEEP S 45, AQUA KEEP AI MI, AQUA KEEP AI M3, AQUA KEEP HP 200, NORSOCRYL S 35, NORSOCRYL FX 007 (Arkema) ; AQUA KEEP 10SH-NF, AQUA KEEP J-550 (Kobo); LUQUASORB CF, LUQUASORB MA 1110, LUQUASORB MR 1600, HYSORB C3746-5 (BASF) ; COVAGEL (Sensient technologies), SANWET IM-5000D (Hoechst Celanese) ;
- les polyacrylates greffés d'amidon vendus sous les dénominations SANWET IM-100, SANWET IM-3900, SANWET IM-5000S (Hoechst) ;
- les copolymères acrylamide/acide acrylique greffés d'amidon sous forme de sel de sodium ou de potassium vendus sous les dénominations WATERLOCK A- 100, WATERLOCK A-200, WATERLOCK C-200, WATERLOCK D-200, WATERLOCK B-204 (Grain Processing Corporation) ;
- les copolymères acrylamide/acide acrylique sous forme de sel de sodium vendus sous la dénomination WATERLOCK G-400 (Grain Processing Corporation) ;
- la carboxyméthylcellulose vendue sous la dénomination AQUASORB A250 (Aqualon) ;
- le polyglutamate de sodium réticulé vendu sous la dénomination GELPROTEIN (Idemitsu Technofine).

Les polymères super-absorbants, en particulier les polymères super-absorbants (polyélectrolytes) qui contiennent des ions alcalins tels que des ions sodium ou potassium, par exemple de type poly(méth)acrylate de sodium ou de potassium, confèrent de nombreuses propriétés aux gels de décontamination selon l'invention.

Ils influencent tout d'abord la rhéologie du produit, notamment son seuil d'écoulement. En termes de mise en œuvre du procédé, l'intérêt des polymères super-absorbants est de garantir une tenue parfaite du gel sur les matériaux traités, notamment sur les surfaces verticales et en surplomb lorsque l'épaisseur de gel pulvérisé est supérieure à 1 mm.

Dans le cadre d'un procédé de décontamination par gel, le polymère super-absorbant est particulièrement intéressant car il absorbe par liaison hydrogène une partie de la solution contenue dans le gel. Le nombre de liaisons hydrogènes formées entre la solution du gel et le polymère super-absorbant tel que le polyacrylate de sodium étant fonction de la charge saline, des phénomènes d'absorption/désorption apparaissent lorsque la charge saline du gel de décontamination est modifiée.

Ce mécanisme est alors particulièrement intéressant lorsqu'il s'agit de décontaminer des matériaux minéraux et poreux comme les matrices cimentaires par exemple.

En effet, au contact du matériau, la charge saline du gel augmente du fait de la présence de particules minérales très souvent à base de calcium. Au sein du polymère super-absorbant tel que le polyacrylate de sodium, la substitution du contre-ion $Na^+$ par $Ca^{2+}$ issu du calcium génère instantanément un phénomène de re-larguage de solution, par exemple de solution biocide, en raison de l'encombrement stérique plus important de l'ion calcium.

La quantité de solution libérée par le polymère super-absorbant tel que le polyacrylate de sodium peut alors diffuser instantanément dans la porosité du matériau et pénétrer en profondeur.

Le phénomène de diffusion de l'agent de décontamination vers le cœur du matériau est beaucoup plus limité dans le cas d'un gel ne contenant pas de super-absorbant.

L'ajout de polymère super-absorbant au gel selon l'invention permet donc d'accroître significativement l'efficacité du gel et du procédé selon l'invention en présence de matériaux poreux contaminés en profondeur sur une épaisseur de un à plusieurs millimètres, par exemple jusqu'à 2, 5, 10, 20 voire 100 mm.

Le polymère super-absorbant peut être choisi de préférence parmi les gammes Aquakeep® ou Norsocryl® commercialisées par la société ARKEMA.

Le gel peut contenir éventuellement, en outre, au moins un agent tensio-actif (à savoir un seul agent tensio-actif ou un mélange d'agents tensio-actifs), de préférence ce ou ces agent(s) tensio-actif(s) est (sont) choisi(s) parmi la famille des agents tensio-actifs non ioniques tels que les copolymères blocs, séquencés comme les copolymères séquencés d'oxyde d'éthylène et d'oxyde de propylène, et les acides gras éthoxylés ; et leurs mélanges.

Pour ce type de gel, les agents tensio-actifs sont de préférence des copolymères blocs commercialisés par la société BASF sous la dénomination PLURONIC®.

Les Pluronics® sont des copolymères séquencés d'oxyde d'éthylène et d'oxyde de propylène.

Ces agents tensio-actifs influencent les propriétés rhéologiques du gel, notamment le caractère thixotropique du produit et son temps de reprise et évitent l'apparition de coulure.

Les agents tensio-actifs permettent, par ailleurs, de maîtriser l'adhésion du déchet sec, et de contrôler la taille des paillettes de résidu sec pour garantir la non-pulvérulence du déchet.

Le solvant est choisi parmi les mélanges d'eau dans une proportion de 40% à 56 % en masse, par exemple 40%, 42,5%, ou 56% en masse, et d'éthanol, dans une proportion de 24% à 42,5% en masse, de préférence de 24% à 40% en masse, par exemple 24%, 40%, ou 42,5% en masse par rapport à la masse du gel.

La quantité d'eau et la quantité d'éthanol sont telles que, lorsqu'elles sont ajoutées aux quantités des composants de la solution colloïdale autres que le solvant (que ces composants soient des composants obligatoires ou éventuels cités plus haut, ou encore d'autres composants additionnels optionnels cités ou non cités), la quantité totale de tous les composants de la solution colloïdale est de 100% en masse.

Un gel particulièrement préféré selon l'invention comprend 42,5% en masse d'eau 42,5% en masse d'éthanol et 15% en masse de $TiO_2$ par rapport à la masse du gel. Le pH de ce gel peut être de 5,5 à 9, par exemple de 5,5 ou 9.

Un autre gel particulièrement préféré selon l'invention comprend 56 % en masse d'eau 24 % en masse d'éthanol et 20% en masse de $TiO_2$ par rapport à la masse du gel. Le pH de ce gel peut être de 5.

Encore un autre gel particulièrement préféré selon l'invention comprend 40% en masse d'eau 40% en masse d'éthanol et 20 % en masse de $TiO_2$ par rapport à la masse du gel. Le pH de ce gel peut être de 5.

Comme on l'a déjà mentionné, il a été montré de manière surprenante, que l'addition d'éthanol dans le gel permet également d'améliorer l'efficacité réductrice du gel, par exemple le taux de réduction du chrome hexavalent.

L'invention concerne, en outre, un procédé de décontamination d'au moins une surface d'un substrat en un matériau solide, ladite surface étant contaminée par au moins une espèce contaminante se trouvant sur ladite surface et éventuellement sous ladite surface (en subsurface) dans la profondeur du substrat, dans lequel on réalise au moins un cycle comprenant les étapes successives suivantes :

a) on applique le gel selon l'invention tel que décrit plus haut sur ladite surface par exemple par pulvérisation, au pinceau ou avec une taloche;

b) on maintient le gel sur la surface au moins pendant une durée suffisante pour que le gel absorbe l'espèce contaminante, puis pour que l'espèce contaminante soit adsorbée à la surface des particules de $TiO_2$, et pour que le gel sèche et forme un résidu sec et solide contenant ladite espèce contaminante adsorbée à la surface des particules de $TiO_2$;

c) on élimine le résidu sec et solide contenant ladite espèce contaminante adsorbée dans le gel à la surface des particules de $TiO_2$, par exemple le résidu sec et solide est éliminé de la surface solide par brossage et/ou aspiration.

Lorsque l'espèce contaminante se trouve sur la surface, on parle de contamination en surface ou de contamination surfacique et par voie de conséquence de décontamination de/en surface ou de décontamination surfacique.

Lorsque l'espèce contaminante se trouve sous ladite surface dans la profondeur du substrat, on parle de contamination de/en subsurface ou de contamination subsurfacique et par voie de conséquence de décontamination de/en subsurface ou de décontamination subsurfacique.

Le procédé selon l'invention permet la décontamination en subsurface de matériaux poreux .

Cependant, le procédé selon l'invention ne permet pas de décontamination en grande profondeur mais seulement sur une profondeur pouvant atteindre au moins quelques microns, généralement sur une profondeur pouvant aller jusqu'à 5 microns à partir de la surface du substrat et avantageusement sur une profondeur pouvant aller jusque 2 microns à partir de la surface du substrat.

Il est à noter que, dans le cas d'une surface non poreuse, la contamination, par exemple la contamination biologique, « inactivée », qui est uniquement une contamination surfacique, est récupérée par les paillettes de gel sec.

Par contre, dans le cas d'une contamination profonde, comme c'est le cas dans les matériaux poreux tels que les matrices cimentaires, le gel sec contiendra le résidu de contamination surfacique et le résidu de contamination subsurfacique (résidu de la contamination se trouvant jusqu'à quelques microns à partir de la surface).

Le substrat solide peut être un substrat poreux, de préférence un substrat minéral poreux. Toutefois, l'efficacité du gel et du procédé selon l'invention est tout aussi bonne en présence d'une surface non poreuse et/ou non minérale.

Avantageusement, le substrat est en au moins un matériau solide choisi parmi les métaux et les alliages métalliques comme l'acier inoxydable, les aciers peints, l'aluminium, et le plomb ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et matériaux cimentaires ; les mortiers et bétons ; les plâtres ; les briques ; la pierre naturelle ou artificielle ; les céramiques.

L'espèce contaminante peut être choisie notamment parmi les espèces contaminantes ioniques, chimiques, biologiques, nucléaires ou radioactives.

Le gel et le procédé selon l'invention permettent l'élimination de l'espèce contaminante quelle que soit cette espèce, qu'elle soit organique ou minérale, liquide ou solide; ou quelle que soit la forme de cette espèce contaminante : que ce contaminant soit sous une forme massive ou particulaire, contenu dans une couche de surface du matériau de la pièce, sous la forme d'un film ou contenu dans un film, par exemple un film de graisses à la surface de la pièce, ou tout simplement déposé sur la surface de la pièce.

Selon la nature de la contamination, le mode d'action du gel selon l'invention diffère : solubilisation de la pellicule contaminante, par exemple de graisses (avec un gel basique par exemple), ou encore adsorption puis inactivation et/ou dégradation, et/ou réduction, et/ou destruction (par photocatalyse) *in situ* des contaminants notamment chimiques ou biologiques dans le cas d'espèces pathogènes (anthrax).

Par gel de décontamination ionique, on entend tout gel, qui lorsqu'il est mis en contact avec une espèce ionique, et notamment une espèce ionique toxique, est susceptible d'extraire cette espèce et de réduire sa toxicité. Cette réduction de la toxicité, se fait généralement par réduction de l'espèce ionique.

Cette espèce ionique peut être choisie parmi les ions métalliques monovalents et multivalents, notamment parmi les ions métalliques monovalents et multivalents toxiques comme les ions chrome (VI), nickel (II), argent (I), cadmium (II), mercure (II), arsenic (III) et plomb (II).

Le procédé selon l'invention, utilisant le gel selon l'invention peut ainsi être mis en œuvre pour décontaminer une surface lisse contaminée par des ions polluants. Ces ions polluants sont éliminés de la surface par absorption au sein du gel, sans attaque chimique de la surface, puis adsorption à la surface des particules de $TiO_2$.

Le procédé selon l'invention, utilisant le gel selon l'invention peut ainsi être mis en œuvre pour décontaminer une surface poreuse contaminée par des ions polluants. Ces ions polluants sont éliminés de la surface par absorption au sein du gel, sans attaque chimique de la surface, puis adsorption à la surface des particules de $TiO_2$.

Par gel de décontamination biologique que l'on peut aussi qualifier de gel biocide, on entend tout gel, qui lorsqu'il est mis en contact avec une espèce biologique, et notamment une espèce biologique toxique, est susceptible d'inactiver ou de détruire celle-ci, ou de réduire sa toxicité.

Par espèce biologique, on entend tout type de micro-organisme tel que les bactéries, les champignons, les levures, les virus, les toxines, les spores notamment les spores de *Bacillus anthracis,* les prions, et les protozoaires.

Les espèces biologiques qui sont éliminées, détruites, inactivées, par le gel selon l'invention sont essentiellement des espèces biotoxiques telles que les spores pathogènes comme par exemple les spores de *Bacillus anthracis,* les toxines comme par exemple la toxine botulique ou la ricine, les bactéries comme les bactéries *Yersinia pestis* et les virus comme le virus de la vaccine ou les virus des fièvres hémorragiques par exemple de type Ebola.

Par gel de décontamination chimique, on entend tout gel qui lorsqu'il est mis en contact avec une espèce chimique, et notamment une espèce chimique toxique, est susceptible de détruire ou d'inactiver celle-ci, ou de réduire sa toxicité. Cette espèce chimique est généralement une molécule organique.

Les espèces chimiques qui sont éliminées par le gel selon l'invention sont notamment les espèces chimiques toxiques telles que les gaz toxiques, en particulier neurotoxiques ou vésicants.

Ces gaz toxiques sont notamment des composés organophosphorés, parmi lesquels on peut citer le Sarin ou agent GB, le VX, le Tabun ou agent GA, le Soman, le Cyclosarin, le diisopropyl fluoro phosphonate (DFP), l'Amiton ou agent VG, le Parathion. D'autres gaz toxiques sont le gaz moutarde ou agent H ou agent HD, la Lewisite ou agent L, l'agent T.

Les espèces nucléaires, radioactives qui peuvent être éliminées, par le gel selon l'invention peuvent être choisies par exemple parmi les oxydes et hydroxydes métalliques notamment sous la forme de précipités solides.

Il est à noter que dans le cas des espèces radioactives, on ne parle pas de destruction ou d'inactivation mais seulement de transfert de la contamination nucléaire, ionique ou sous forme solide, vers les paillettes de gel sec.

Avantageusement, le gel est appliqué sur la surface à décontaminer à raison de 100 g à 2000 g de gel par $m^2$ de surface, de préférence de 500 à 1500 g de gel par $m^2$ de surface, de préférence encore de 600 à 1000 g de gel par $m^2$ de surface, ce qui correspond généralement à une épaisseur de gel déposé sur la surface de 0,1 mm à 2 mm, de préférence de 0,5 mm à 2 mm, de préférence encore de 1 mm à 2 mm.

Avantageusement, le gel est appliqué sur la surface solide par pulvérisation, au pinceau ou avec une taloche.

Avantageusement, pendant tout ou partie de l'étape a), et/ou pendant tout ou partie de l'étape b), de préférence pendant toute la durée de l'étape b), le gel maintenu sur la surface est exposé à un rayonnement visible ou à un rayonnement Ultraviolet A, B, ou C (UVA, UVB, ou UVC), ou à un autre rayonnement, de manière à inactiver, et/ou dégrader, et/ou réduire, et/ou détruire, l'espèce contaminante par photocatalyse.

En effet, une exposition du gel à un autre rayonnement que le rayonnement visible ou un rayonnement Ultraviolet A, B, ou C, pourrait être envisagée, notamment si le $TiO_2$ est dopé par un élément chimique adapté.

Il est possible de n'exposer le gel à un rayonnement (irradier le gel) que pendant une partie de l'étape b) ce qui permet de moduler l'effet photocatalytique. En effet, l'effet photocatalytique ne se produit alors que pendant la durée de l'irradiation.

En exposant le gel déposé sur la surface à un rayonnement visible, et de préférence à un rayonnement ultraviolet, ou à un autre rayonnement, on met à profit l'effet photocatalytique du $TiO_2$ contenu dans le gel selon l'invention pour réaliser la décontamination, par exemple, pour réduire des ions ou pour dégrader des espèces contaminantes chimiques, telles que des molécules organiques, ou des espèces contaminantes biologiques.

Le résidu sec a une toxicité moindre par rapport à la pollution, contamination initiale, dans le cas d'une pollution causée par une espèce contaminante ionique réductible, telle qu'un ion multivalent.

En effet, l'espèce contaminante est réduite, sous rayonnement, à cause de l'effet photocatalytique des particules de $TiO_2$ constitutives des paillettes de gel sec, en une espèce réduite possédant un degré d'oxydation tel que la toxicité de l'espèce réduite est moindre que celle de l'espèce contaminante initiale.

De la même manière, le résidu sec a une toxicité moindre par rapport à la pollution, contamination initiale, dans le cas d'une pollution causée par une espèce contaminante chimique telle qu'une molécule organique.

En effet, l'espèce contaminante est oxydée, sous rayonnement, à cause de l'effet photocatalytique des particules de $TiO_2$ constitutives des paillettes de gel sec, en une ou plusieurs molécules dont la toxicité est moindre que celle de l'espèce contaminante initiale.

Encore de la même manière, le résidu sec a une toxicité moindre par rapport à la pollution, contamination, initiale, dans le cas d'une pollution causée par une espèce contaminante biologique telle qu'un microorganisme.

En effet, le microorganisme est inactivé, sous rayonnement à cause de l'effet photocatalytique des particules de $TiO_2$ constitutives des paillettes de gel sec, en un produit biologique dont la toxicité est moindre que celle de l'espèce contaminante initiale.

En d'autres termes, de manière générale les propriétés photocatalytiques du gel permettent d'activer comme on le souhaite la dégradation/diminution de la toxicité de l'espèce contaminante.

Avantageusement (lors de l'étape b)), le séchage est réalisé à une température de 1°C à 50°C, de préférence de 15°C à 25°C, et sous une humidité relative de 20% à 80%, de préférence de 20% à 70%.

Avantageusement, le gel est maintenu sur la surface pendant une durée de 2 à 72 heures, de préférence de 2 à 48 heures, de préférence encore de 4 à 24 heures.

Avantageusement, le résidu sec et solide se présente sous la forme de particules, par exemple de paillettes, d'une taille de 1 à 10 mm, de préférence de 2 à 5 mm.

Avantageusement, le résidu sec et solide est éliminé de la surface solide par brossage et/ou aspiration.

Selon l'invention, le résidu de gel sec contenant l'espèce contaminante, confine cette espèce contaminante, notamment dans le cas d'une espèce contaminante ionique, grâce à la complexation chimique par adsorption des ions en surface des particules de $TiO_2$ constitutives du résidu de gel sec, généralement des paillettes de gel sec. Grâce à cette adsorption de l'espèce contaminante, il n'y a pas de relargage de l'espèce contaminante, notamment ionique en cas de lixiviation du résidu de gel sec, notamment sous forme de paillettes.

Avantageusement, le cycle décrit plus haut peut être répété par exemple de 1 à 10 fois en utilisant le même gel lors de tous les cycles ou en utilisant des gels différents lors d'un ou de plusieurs cycle(s).

Avantageusement, lors de l'étape b), le gel, avant séchage total, est remouillé avec un solvant, de préférence avec le solvant du gel appliqué lors de l'étape a) ce qui évite alors généralement de répéter l'application du gel sur la surface et occasionne une économie de réactif et une quantité de déchets limitée. Cette opération de remouillage peut être répétée par exemple de 1 à 10 fois.

Le procédé selon l'invention possède toutes les propriétés avantageuses inhérentes au gel de décontamination qu'il met en œuvre, essentiellement dues au $TiO_2$ contenu dans le gel, et qui ont déjà été largement exposées plus haut.

En résumé, le procédé et le gel selon l'invention présentent notamment les propriétés avantageuses suivantes :

- l'application du gel par pulvérisation,
- l'adhérence aux parois et aux plafonds,
- l'obtention de l'efficacité maximale de décontamination à l'issue de la phase de séchage du gel, y compris en situation de contamination pénétrante notamment dans le cas de surfaces poreuses (décontamination subsurfacique : voir plus haut).

En général, on fait en sorte que le temps de séchage soit supérieur ou égal à la durée nécessaire pour l'adsorption et éventuellement l'inactivation et/ou la dégradation et/ou la réduction et/ou la destruction de l'espèce contaminante par photocatalyse, par exposition à un rayonnement.

Dans le cas d'une adsorption et éventuellement d'une inactivation profonde(s), on fait généralement appel à un remouillage.

Autrement dit si l'espèce contaminante doit subir un traitement photocatalytique pour diminuer sa toxicité, on fait en sorte que le temps de séchage soit supérieur ou égal à la durée nécessaire pour l'adsorption et l'inactivation photocatalytique de l'espèce contaminante.

Sinon, si un traitement photocatalytique de l'espèce contaminante n'est pas nécessaire, alors pour diminuer sa toxicité, il suffit de faire en sorte que le temps de séchage soit simplement supérieur ou égal à la durée nécessaire pour l'adsorption.

- le traitement d'une gamme très large de matériaux,
- l'absence d'altération mécanique ou physique des matériaux à l'issue du traitement,
- la mise en œuvre du procédé dans des conditions climatiques variables,
- la réduction du volume de déchets,
- la facilité de récupération du déchet sec.

D'autres caractéristiques et avantages de l'invention apparaîtront mieux à la lecture de la description détaillée qui suit, cette description étant faite à titre illustratif et non limitatif, en liaison avec les dessins joints.

**BREVE DESCRIPTION DES DESSINS**

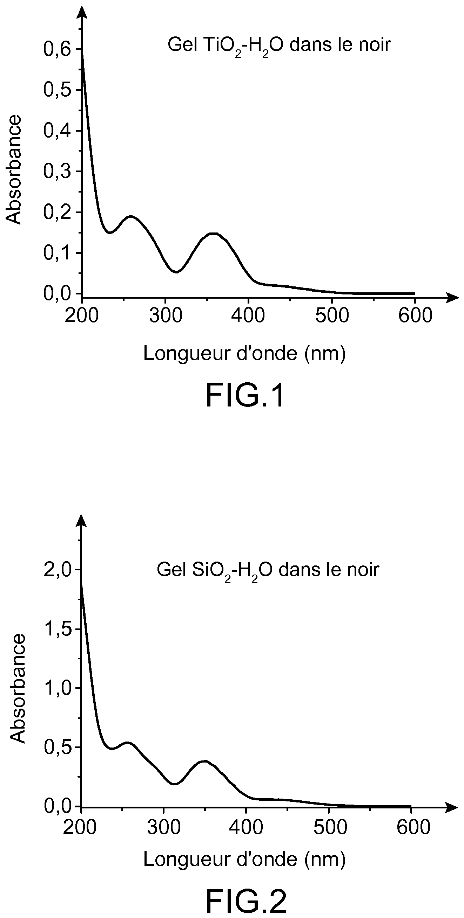

- La Figure 1 est un graphique qui représente le spectre UV-Visible de la solution obtenue, dans l'exemple 3 non conforme à l'invention, suite à la lixiviation des paillettes d'un gel à base de $TiO_2$, à savoir le gel préparé dans l'exemple 1 non conforme à l'invention, dont le solvant est de l'eau et dont l'application et le séchage sont réalisés dans le noir.
  Ce graphique est dénommé « Gel $TiO_2$-$H_2O$ dans le noir ».
  En abscisse est portée la longueur d'onde (en nm), et en ordonnée est portée l'absorbance.
- La Figure 2 est un graphique qui représente le spectre UV-Visible de la solution, obtenue dans l'exemple 3 non conforme à l'invention suite à la lixiviation des paillettes d'un gel comparatif, non conforme à l'invention, à base de $SiO_2$, à savoir le gel préparé dans l'exemple 3, dont le solvant est de l'eau et dont l'application et le séchage sont réalisés dans le noir.
  Ce graphique est dénommé « Gel $SiO_2$-$H_2O$ dans le noir ».
  En abscisse est portée la longueur d'onde (en nm), et en ordonnée est portée l'absorbance.

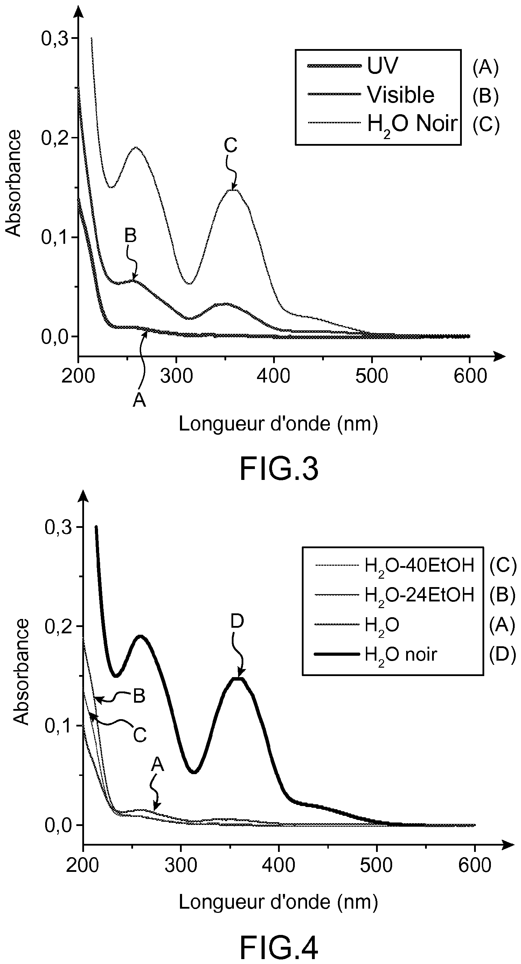

- La Figure 3 est un graphique qui représente le spectre UV-Visible des solutions obtenues suite à la lixiviation des paillettes du gel (« Gel $H_2O$-40 EtOH ») selon l'invention décrit dans l'exemple 5.

  Ces paillettes sont obtenues soit par séchage sous le rayonnement d'une lampe UV (courbe A), soit par séchage sous lumière visible (courbe B).

  On a également porté sur ce graphique, à titre de comparaison, le spectre UV-visible (courbe C) de la solution obtenue suite à la lixiviation des paillettes d'un gel à base de $TiO_2$, à savoir le gel préparé dans l'exemple 1 non conforme à l'invention, dont le solvant est de l'eau et dont l'application et le séchage sont réalisés dans le noir.

  En abscisse est portée la longueur d'onde (en nm), et en ordonnée est portée l'absorbance.

- La Figure 4 est un graphique qui représente le spectre UV-Visible des solutions obtenues suite à la lixiviation des paillettes des gels (« Gel $H_2O$ » (Courbe A), « Gel $H_2O$-24 EtOH » (Courbe B), et « Gel $H_2O$-40 EtOH » (Courbe C)) décrits dans l'exemple 7 (voir Tableau 7).

  Ces paillettes sont obtenues par séchage sous le rayonnement d'une lampe UV.

  On a également porté sur ce graphique, à titre de comparaison, le spectre UV-visible (courbe D) de la solution obtenue suite à la lixiviation des paillettes d'un gel à base de $TiO_2$, à savoir le gel préparé à l'exemple 1 non conforme à l'invention, dont le solvant est de l'eau et dont l'application et le séchage sont réalisés dans le noir.

  En abscisse est portée la longueur d'onde (en nm), et en ordonnée est portée l'absorbance.

- La Figure 5 est un graphique qui représente l'évolution de la viscosité en fonction du taux de cisaillement du « Gel $H_2O$ » (courbe A) et du « Gel $H_2O$-40EtOH » (Courbe B) (Voir exemple 8).

  En abscisse est porté le taux de cisaillement (en s-1) et en ordonnée est portée la viscosité (en Pa.s).

- La Figure 6 est un graphique qui représente l'évolution de la contrainte de cisaillement en fonction de la déformation du « Gel $H_2O$ » (courbe A) et du « Gel $H_2O$-40EtOH » (Courbe B) (voir exemple 8).

  En abscisse est portée la déformation (sans unité), et en ordonnée est portée la contrainte de cisaillement (en Pa).

- La Figure 7 est un graphique qui représente l'évolution de la perte de masse en fonction du temps pour le « Gel $H_2O$ » (Courbe A), et le « Gel $H_2O$-24EtOH » (Courbe B) à 25°C et 50% d'humidité relative (voir exemple 11).

- La Figure 8 est une photographie des paillettes obtenues suite au séchage du « Gel $H_2O$-24EtOH » dans une nacelle de 2 mm de profondeur (voir exemple 11).

- La Figure 9 est un graphique qui représente l'évolution de la viscosité en fonction du taux de cisaillement pour les gels étudiés dans l'exemple 9.

  En abscisse est porté le taux de cisaillement (en $s^{-1}$), et en ordonnée est portée la viscosité (en Pa.s).

- La Figure 10 est un graphique qui représente l'évolution de la contrainte de cisaillement en fonction de la déformation pour les gels étudiés dans l'exemple 10.

  En abscisse est portée la déformation (sans unité), et en ordonnée est portée la contrainte de cisaillement (en Pa).

## EXPOSÉ DÉTAILLÉ DE MODES DE RÉALISATION PARTICULIERS

Le gel selon l'invention peut être facilement préparé à la température ambiante.

Par exemple, le gel selon l'invention peut être préparé en dispersant, de préférence progressivement, la quantité voulue de l'agent(s) viscosant inorganique, à savoir le $TiO_2$, sous la forme de particules, par exemple d'un diamètre moyen de 2 à 200 nm dans le solvant du gel.

Cette dispersion peut être réalisée par agitation mécanique, par exemple au moyen d'un agitateur mécanique équipé d'une hélice à trois pales. La vitesse de rotation est par exemple de 200 tours/minute, et la durée de l'agitation est par exemple de 3 à 5 minutes.

A l'issue de la dispersion on obtient une suspension liquide blanche opaque, dont le pH est généralement d'environ 3,5.

On augmente alors progressivement le pH, toujours sous agitation, à l'aide d'une base, par exemple à l'aide de soude NaOH 0,1 M, jusqu'à ce que le pH soit égal, par exemple à 5, ce qui va permettre de former un gel visqueux blanc opaque.

Pendant l'addition de base, par exemple de soude, on augmente graduellement l'agitation, au fur et à mesure que la viscosité croît, pour arriver à environ 400 à 600 tours/min sans qu'il n'y ait de projections. Le gel est ensuite maintenu sous agitation par exemple pendant 2 à 5 minutes, de manière à obtenir un gel parfaitement homogène.

On note ici que la quantité de base, telle que de la soude, ajoutée peut être considérée comme négligeable par rapport à la quantité de solvant initiale, on peut ainsi considérer que la composition massique initiale en $TiO_2$ et en solvant reste valable une fois que l'on est à pH = 5.

Le pH du gel ainsi préparé est donc faiblement acide car égal à 5.

En continuant d'augmenter le pH, jusqu'à un pH égal à 7, on peut obtenir un gel neutre conforme à l'invention qui permet ainsi d'éviter l'attaque chimique des surfaces traitées, et même un gel faiblement basique conforme à l'invention, à un pH faiblement basique de plus de 7 à moins de 9, ou encore un gel très basique conforme à l'invention, à un pH très basique de 9 ou plus qui possède donc un pouvoir dégraissant mais qui n'attaque pas les surfaces traitées.

Dans le gel neutre et le gel faiblement basique, mais aussi dans le gel très basique, la quantité de soude peut généralement toujours être considérée comme négligeable.

Généralement, le gel selon l'invention doit présenter une viscosité inférieure à 200 mPa.s sous un cisaillement de 1000 s$^{-1}$ de manière à permettre la pulvérisation sur la surface à décontaminer, à distance (par exemple à une distance de 1 à 5 m) ou à proximité (par exemple à une distance inférieure à 1 m, de préférence de 50 à 80 cm).

Le temps de reprise de la viscosité doit généralement être inférieur à une seconde et la viscosité sous faible cisaillement supérieure à 10 Pa.s pour ne pas couler sur une paroi verticale.

Le gel selon l'invention ainsi préparé est ensuite appliqué sur la surface solide à décontaminer d'un substrat ou d'un matériau solide, en d'autres termes sur la surface ayant été exposée à une contamination par exemple à une contamination biologique.

Cette contamination a déjà été décrite plus haut. En particulier, la contamination biologique peut être constituée par une ou plusieurs des espèces biologiques déjà définies plus haut.

Comme on l'a déjà indiqué plus haut, dans le gel selon l'invention, le $TiO_2$ joue, outre le rôle d'agent viscosant inorganique, le rôle d'agent actif de décontamination, par exemple d'agent actif de décontamination biologique, permettant d'éliminer, de détruire, ou d'inactiver une espèce polluante, contaminante, par exemple une espèce biologique contaminante.

Il n'existe aucune limitation quant au matériau qui constitue la surface à décontaminer, en effet le gel selon l'invention permet de traiter sans aucun endommagement, toutes sortes de matériaux même fragiles.

Même des surfaces en des matériaux tels les alliages de métaux légers de type aluminium qui ne pouvaient être traitées sans être détériorées avec les gels de l'art antérieur peuvent être traitées avec succès avec les gels selon l'invention.

Le gel selon l'invention ne génère aucune altération, érosion, attaque, chimique, mécanique ou physique du matériau traité. Le gel selon l'invention n'est donc en aucune manière préjudiciable à l'intégrité des matériaux traités et permet même leur réutilisation. Ainsi, des matériels sensibles tels que des équipements militaires sont préservés et pourront après leur décontamination être réutilisés, tandis que les monuments traités par le gel selon l'invention ne sont absolument pas dégradés et voient leur intégrité visuelle et structurale conservée.

Ce matériau du substrat peut donc être choisi parmi par exemple les métaux et alliages comme l'acier inoxydable, l'aluminium, et le plomb; les polymères tels que les matières plastiques ou caoutchoucs parmi lesquels on peut citer les PVC, PP, PE notamment HDPE, PMMA, PVDF, PC; les verres ; les ciments et les matériaux cimentaires ; les mortiers et bétons ; les plâtres ; les briques ; la pierre naturelle ou artificielle ; les céramiques.

Dans tous les cas, quel que soit le matériau, l'efficacité de décontamination par le gel selon l'invention est totale.

La surface traitée peut être peinte ou non peinte.

Le gel selon l'invention est aussi efficace sur les matériaux poreux tels que les matrices cimentaires comme les pâtes, les mortiers et les bétons, les briques, les plâtres, ou encore la pierre naturelle ou artificielle.

L'efficacité du traitement avec le gel selon l'invention est généralement totale, y compris sur les matériaux contaminés sur plusieurs microns de profondeur (voir plus haut).

Il n'existe également aucune limitation quant à la forme, la géométrie et la taille de la surface à décontaminer, le gel selon l'invention et le procédé le mettant en œuvre permettent le traitement de surfaces de grande taille, de géométries complexes, présentant par exemple des creux, angles, recoins.

Le gel selon l'invention assure le traitement efficace non seulement de surfaces horizontales telles que des planchers, mais aussi de surfaces verticales telles que des murs, parois, ou de surfaces inclinées ou en surplomb telles que des plafonds.

Par rapport aux procédés de décontamination, par exemple des procédés de décontamination biologiques existants qui mettent en œuvre des liquides tels que des solutions, le procédé de décontamination selon l'invention qui met en œuvre un gel est particulièrement avantageux pour le traitement de matériaux de grande surface, non transportables et implantés à l'extérieur. En effet, le procédé selon l'invention du fait de la mise en œuvre d'un gel, permet la décontamination *in situ* en évitant l'épandage de solutions chimiques dans l'environnement et la dispersion des espèces contaminantes.

Le gel selon l'invention peut être appliqué sur la surface à traiter par tous les procédés d'application connus de l'homme du métier.

Des procédés classiques sont la pulvérisation par exemple au pistolet ou l'application au moyen d'un pinceau, ou d'une taloche.

Pour l'application par pulvérisation du gel selon l'invention sur la surface à traiter, la solution colloïdale peut par exemple être véhiculée par l'intermédiaire d'une pompe basse pression, par exemple une pompe qui met en œuvre une pression inférieure ou égale à 7 bars, soit environ $7.10^5$ Pascals.

L'éclatement du jet de gel sur la surface peut être obtenu par exemple au moyen d'une buse à jet plat ou à jet rond.

La distance entre la pompe et la buse peut être quelconque, par exemple elle peut être de 1 à 50 m, notamment de 1 à 25 m.

Le temps de reprise de la viscosité suffisamment court des gels selon l'invention, permet aux gels pulvérisés d'adhérer à toutes les surfaces, par exemple à des parois.

La quantité de gel déposée sur la surface à traiter est généralement de 100 à 2000 g/m$^2$, de préférence de 500 à 1500 g/m$^2$, de préférence encore de 600 à 1000 g/m$^2$.

La quantité de gel déposée par unité de surface et, par voie de conséquence, l'épaisseur du gel déposé influence la vitesse de séchage.

Ainsi, lorsque l'on pulvérise un film, couche de gel d'une épaisseur de 0,5 mm à 2 mm sur la surface à traiter, le temps de contact efficace entre le gel et la surface est alors équivalent à son temps de séchage, période pendant laquelle le principe actif, ici le TiO$_2$, contenu dans le gel va interagir avec la contamination.

Dans le cas des substrats poreux, par exemple des matrices cimentaires, le temps d'action de la solution de décontamination, par exemple de la solution biocide, ayant pénétré dans le cœur du matériau peut être supérieur au temps de séchage du gel, auquel cas il est généralement nécessaire soit de réaliser un remouillage avec la solution de décontamination, soit de répéter une pulvérisation du gel.

En outre, il a été montré de manière surprenante que la quantité de gel déposée lorsqu'elle se situe dans les plages mentionnées plus haut et en particulier lorsqu'elle est supérieure ou égale à 500 g/m$^2$ et notamment dans la plage de 500 à 1500 g/m$^2$, ce qui correspond à une épaisseur minimale de gel déposée par exemple supérieure ou égale à 500 $\mu$m pour une quantité de gel déposée supérieure ou égale à 500 g/m$^2$, permettait après séchage du gel d'obtenir une fracturation du gel sous la forme de paillettes millimétriques, par exemple d'une taille de 1 à 10 mm, de préférence de 2 à 5 mm aspirables.

La quantité de gel déposée et donc l'épaisseur de gel déposée, de préférence supérieure ou égale à 500 g/m$^2$ soit 500 $\mu$m, de préférence encore supérieure ou égale à 1000 g/m$^2$ soit 1000 $\mu$m (1 mm) est le paramètre fondamental qui influence la taille des résidus secs formés après séchage du gel et qui assure ainsi que des résidus secs de taille millimétrique et non des résidus pulvérulents soient formés, de tels résidus étant facilement éliminés par un procédé mécanique et de préférence par aspiration.

Le gel est ensuite maintenu sur la surface à traiter pendant toute la durée nécessaire à son séchage. Au cours de cette étape de séchage dont on peut considérer qu'elle constitue la phase active du procédé selon l'invention, le solvant contenu dans le gel, par exemple l'eau contenue dans le gel s'évapore jusqu'à l'obtention d'un résidu sec et solide.

La durée de séchage dépend de la composition du gel dans les gammes de concentration de ses constituants données plus haut, mais aussi, comme on l'a déjà précisé, de la quantité de gel déposée par unité de surface c'est-à-dire de l'épaisseur de gel déposée.

La durée de séchage dépend aussi des conditions climatiques à savoir de la température et de l'humidité relative de l'atmosphère dans laquelle se trouve la surface solide.

Le procédé selon l'invention peut être mis en œuvre dans des conditions climatiques extrêmement larges, à savoir à une température T de 1°C à 50°C et à une humidité relative HR de 20% à 80%.

La durée de séchage du gel selon l'invention est donc généralement de 1 heure à 24 heures à une température T de 1°C à 50°C et à une humidité relative HR de 20% à 80%. Le gel appliqué sur la surface contaminée peut être exposé à un rayonnement lumineux pendant la durée du séchage.

Le rayonnement lumineux peut être un rayonnement visible ou un rayonnement ultraviolet A, B, ou C, produit par exemple par une lampe à UV. Ainsi, le gel appliqué sur la surface peut être exposé à un rayonnement UV d'une longueur d'onde de 365 nm.

La durée de l'exposition au rayonnement qui correspond à la durée du séchage est généralement de 1 à 24h. Le séchage peut être accéléré par cette exposition aux UV.

L'espèce contaminante présente sur la surface est, dans un premier temps, absorbée au sein du gel par solubilisation, diffusion et adsorption en surface des particules de TiO$_2$.

C'est ensuite le pouvoir photocatalytique du TiO$_2$ qui va permettre la destruction et/ou l'inactivation et/ou la dégradation de l'espèce contaminante, par exemple la réduction d'un élément multivalent (passage par exemple du chrome hexavalent au chrome trivalent), la dégradation d'une espèce contaminante chimique (molécule organique), ou encore l'activation du pouvoir biocide du TiO$_2$, tout au long du séchage.

A l'issue du séchage, l'espèce contaminante est détruite et/ou inactivée et/ou dégradée et/ou absorbée, et/ou adsorbée.

Notamment, la toxicité des espèces contaminantes est drastiquement voire totalement annihilée.

Après séchage du gel, la contamination, par exemple la contamination biologique inactivée est éliminée lors de la récupération du résidu de gel sec décrite plus bas.

A l'issue du séchage du gel, le gel se fracture de manière homogène pour donner des résidus secs solides millimétriques, par exemple d'une taille de 1 à 10 mm, de préférence de 2 à 5 mm non pulvérulents, généralement sous la forme de paillettes solides.

Les résidus secs peuvent contenir la ou les espèce(s) contaminante(s) inactivée(s).

Les résidus secs, tels que des paillettes, obtenus à l'issue du séchage présentent une faible adhérence à la surface du matériau décontaminé. De ce fait, les résidus secs obtenus après séchage du gel peuvent être facilement récupérés par simple brossage et/ou aspiration. Toutefois, les résidus secs peuvent aussi être évacués par jet de gaz, par exemple par jet d'air comprimé.

Ainsi, aucun rinçage par un liquide n'est généralement nécessaire, et le procédé selon l'invention ne génère aucun effluent secondaire.

On peut cependant, bien que cela ne soit pas préféré, et si on le souhaite, éliminer les résidus secs au moyen d'un jet de liquide.

Le procédé selon l'invention réalise donc ainsi tout d'abord une importante économie de réactifs chimiques par rapport à un procédé de décontamination par lavage avec une solution. Ensuite du fait qu'un déchet sous la forme d'un résidu sec directement aspirable est obtenu, une opération de rinçage avec de l'eau ou avec un liquide, généralement nécessaire à l'élimination des traces d'agents chimiques de la pièce, est généralement évitée. Il en résulte bien évidemment une diminution de la quantité d'effluents produits mais aussi une simplification notable en termes de filière de traitement des déchets et d'exutoire.

En raison de la composition majoritairement minérale du gel selon l'invention et de la faible quantité de déchets produits, le déchet sec peut être stocké ou dirigé vers une filière d'évacuation (« exutoire ») sans traitement préalable.

A l'issue du procédé selon l'invention, on récupère un déchet solide sous forme de paillettes pouvant être conditionnées en l'état, directement conditionnables, il résulte comme on l'a déjà indiqué plus haut une diminution significative de la quantité d'effluents produits ainsi qu'une simplification notable en terme de filière de traitement du déchet et d'exutoire.

De plus, dans le domaine du nucléaire, le fait de ne pas avoir à retraiter les paillettes avant le conditionnement du déchet constitue un atout considérable ; cela autorise l'utilisation d'agent actifs performants jusqu'ici interdits dans les liquides de décontamination en raison des contraintes d'exploitation des stations de traitement des effluents liquides (« STEL ») .

A titre d'exemple, dans le cas courant où l'on applique 1000 grammes de gel par $m^2$ de surface traitée, la masse de déchet sec produite est inférieure à 200 grammes par $m^2$.

L'invention va maintenant être décrite en référence aux exemples suivants, donnés à titre illustratif et non limitatif.

**Exemples** :

Exemple 1 non conforme à l'invention:

Dans cet exemple, on décrit les gels faiblement acides, neutres, ou basiques à base de $TiO_2$, utilisés dans les exemples 2, 3, 4, 6 et 7

Le gel faiblement acide à base de $TiO_2$ est un gel dont la composition est la suivante en pourcentages massiques :

- 20% de $TiO_2$;
- 80% d'eau distillée.

Le $TiO_2$ est le $TiO_2$ commercialisé par la société Aerosil® sous la dénomination Degussa P25 ®.

Ce gel est préparé de la manière suivante :
Dans un premier temps, les particules de $TiO_2$ sont dispersées dans de l'eau à l'aide d'un agitateur mécanique, muni d'un agitateur à trois pales, à une vitesse de 200 tours/min.

On obtient ainsi une suspension liquide blanche opaque dont le pH est d'environ 3,5.

On augmente alors progressivement le pH, toujours sous agitation, à l'aide de soude NaOH 0,1 M, jusqu'à ce que le pH soit égal à 5, ce qui va permettre de former un gel visqueux blanc opaque.

Pendant l'addition de soude, on augmente graduellement l'agitation, au fur et à mesure que la viscosité croît, pour arriver à environ 400 à 600 tours/min sans qu'il n'y ait de projections. Le gel est ensuite maintenu sous agitation pendant 5 minutes.

On note ici que la quantité de soude ajoutée est systématiquement négligeable par rapport à la quantité d'eau initiale, on peut ainsi considérer que la composition massique de 20% de $TiO_2$ et 80% de $H_2O$ reste valable une fois que l'on est à pH = 5.

Le pH du gel ainsi préparé est donc faiblement acide car égal à 5.

En continuant d'augmenter le pH, jusqu'à un pH égal à 7, on peut obtenir un gel neutre qui permet ainsi d'éviter l'attaque chimique des surfaces traitées, et même un gel très basique à un pH supérieur ou égal à 9, conforme à l'invention qui possède donc un pouvoir dégraissant.

Dans le gel neutre et le gel très basique, la quantité de soude peut toujours être considérée comme négligeable.

Exemple 2 non conforme à l'invention:

Dans cet exemple, on montre l'efficacité de la décontamination de surfaces lisses à l'aide du gel préparé dans l'exemple 1 non conforme à l'invention, qui contient du $TiO_2$.

L'essai de décontamination est réalisé sur des surfaces lisses polluées avec du Cr(VI) à l'aide du gel à base de $TiO_2$, préparé dans l'exemple 1 non conforme à l'invention.

Les surfaces lisses sont une surface en céramique, une surface en aluminium, et une surface en polyéthylène haute densité (HDPE).

Pour réaliser l'essai sur la surface lisse en céramique constituée par un carreau en céramique, 0,25mL d'une solution de $K_2Cr_2O_7$ à $10^{-2}$M (de chez Sigma-Aldrich® avec une pureté $\geq$ 99.0%, dissous dans $H_2O$), équivalente à une solution concentrée à $2.10^{-2}$M en Cr(VI), sont déposés sur le carreau en céramique.

On laisse ensuite sécher la goutte pendant toute une nuit. On obtient alors une tâche jaune, sur laquelle le gel acide (pH = 5) préparé dans l'exemple 1 non conforme à l'invention est déposé, appliqué, dans le noir avec une épaisseur de 1,25 mm.

On laisse ensuite sécher le gel dans le noir afin de stopper toute éventuelle réduction par photocatalyse du Cr(VI) par le $TiO_2$, et pour n'observer que le phénomène de sorption au sein du gel. Après 4h30 de séchage, les paillettes de gel sec sont finalement récupérées par brossage. La tâche jaune a alors totalement disparu du support, et les paillettes du gel se sont colorées en jaune (qui est la couleur caractéristique du chrome hexavalent), preuve que le chrome a bien été absorbé au sein des paillettes.

Les paillettes sont ensuite dissoutes dans de l'acide fluorhydrique pur, sous agitation mécanique, à 80°C, pendant plusieurs heures. La solution obtenue est analysée par spectrométrie à émission optique à plasma induit (« *Inductively Coupled Plasma - Optical Emission Spectroscopy* » ou « *ICP-OES* » en anglais) avec un appareil ThermoFisher Scientific iCAP 6000 Sériés®.

Les résultats obtenus sont présentés dans le Tableau 1 ci-dessous.

On note ici que la masse initiale de chrome hexavalent déposée sur le support est de 0,26 mg.

Tableau 1 : Résultats ICP-OES obtenus suite à la dissolution des paillettes du gel obtenues dans l'exemple 2.

| Masse des paillettes | Volume total de HF | Masse de Chrome déterminée par ICP | % de décontamination |
|---|---|---|---|
| 567 mg | 105 mL | 0,26 mg | 100% |

On observe ainsi que le gel à base de $TiO_2$ décontamine parfaitement le carreau de céramique contaminé par du Cr(VI).

On réalise le même essai de décontamination que sur la surface lisse en céramique, mais cette fois sur une surface lisse en aluminium, et sur une surface lisse en polyéthylène haute densité (HDPE) contaminées par du Cr(VI).

Le protocole expérimental est le même que celui déjà décrit plus haut pour la décontamination de la surface lisse en céramique.

Dans chaque cas, l'ensemble du chrome est capté par les paillettes de gel, conduisant à une efficacité de décontamination de 100%.

Le gel à base de $TiO_2$ permet donc bien la décontamination totale en chrome hexavalent d'une surface lisse quel que soit le matériau constituant cette surface.

Example 3 non conforme à l'invention:

Dans cet exemple, on montre les propriétés de rétention (confinement) de la pollution ionique après décontamination d'une surface par un gel à base de $TiO_2$.

Plus exactement, il s'agit dans cet exemple de démontrer la capacité d'un gel acide (pH = 5) à base de $TiO_2$, tel que décrit dans l'exemple 1 non conforme à l'invention à confiner le chrome hexavalent dans les paillettes de gel séché.

Ces propriétés, cette capacité, de confinement, de rétention, du gel à base de $TiO_2$, sont comparées à celles d'un gel comparatif base de $SiO_2$.

Le gel comparatif, à base de $SiO_2$, est un gel dont la composition est la suivante en pourcentages massiques :

- 12% de silice;
- 88% d'eau distillée.

La silice est la silice commercialisée par la société EVONIK® sous la dénomination Aerosil®.

Le gel comparatif, à base de $SiO_2$, est préparé de la manière suivante :

La silice est ajoutée progressivement dans de l'eau distillée, sous agitation mécanique, à l'aide d'un agitateur mécanique muni d'un agitateur à trois pales, à une vitesse de 200 tours/min. Pendant l'addition de la silice, on augmente graduellement l'agitation, au fur et à mesure que la viscosité croît, pour arriver à environ 400 à 600 tours/min sans qu'il n'y ait de projections. Le gel est ensuite maintenu sous agitation pendant 5 minutes.

Le pH de ce gel est de 4,5.

On réalise ensuite un essai de décontamination avec le gel à base de TiO$_2$. Au début, le protocole opératoire de cet essai est le même que celui observé dans l'exemple 2 non conforme à l'invention, à savoir : 0,25mL d'une solution de K$_2$Cr$_2$O$_7$ à 10$^{-2}$M (de chez Sigma-Aldrich avec une pureté ≥ 99.0%, dissous dans H$_2$O), équivalente à une solution concentrée à 2.10$^{-2}$M en Cr(VI), sont déposés sur un carreau de céramique.

On laisse ensuite sécher la goutte pendant toute une nuit.

On obtient alors une tâche jaune, sur laquelle le gel à base de TiO$_2$ préparé dans l'exemple 1 non conforme à l'invention est déposé, appliqué dans le noir (afin d'éviter la réduction du chrome par photocatalyse), avec une épaisseur de 1,25 mm.

On laisse ensuite sécher le gel dans le noir afin de stopper toute éventuelle réduction par photocatalyse du Cr(VI) par le TiO$_2$ et pour n'observer que le phénomène de sorption au sein du gel.

Après 4h30 de séchage, le protocole suivant est appliqué :

- on récupère puis re-disperse la totalité des paillettes dans 20mL d'H$_2$O sous agitation mécanique à l'aide d'un barreau aimanté pendant 2h. On réalise ainsi une lixiviation des paillettes dans H$_2$O. Théoriquement, si la totalité du chrome présent dans les paillettes est relarguée, on aura en solution [Cr(VI)]$_0$ = 2,5.10$^{-4}$M. Cette valeur de [Cr(VI)]$_0$ = 2,5.10$^{-4}$M est obtenue de la manière suivante : 0,25 mL d'une solution à 2.10$^{-2}$M en Cr(VI) séchée que l'on retrouve finalement dans 20 mL soit : (0,25 x 2.10$^{-2}$)/20 = 2,5.10$^{-4}$.
- la suspension obtenue est ensuite centrifugée pendant 30 minutes à 4400 tours/min, et finalement filtrée à l'aide d'un filtre de 0,22 μm. Ces étapes permettent de séparer complètement les particules de TiO$_2$ et d'obtenir un lixiviat contenant les espèces faiblement liées aux paillettes de gel.
- le lixiviat est analysé à l'aide d'un spectromètre UV-Visible UV-1800 de marque Shimadzu® préalablement étalonné. L'étalonnage a été réalisé à l'aide de différentes solutions de K$_2$Cr$_2$O$_7$ de concentrations connues, et la ligne de base est réalisée sur de l'eau ultrapure. Le spectre d'absorption du chrome hexavalent, en milieu acide, présente alors des pics aux longueurs d'ondes 260 nm et 352 nm.
- le spectre obtenu pour le lixiviat obtenu à partir des paillettes du gel à base de TiO$_2$ est donné sur la La Figure 1.

La loi de Beer-Lambert permet alors d'avoir accès à la concentration en Cr(VI) présent dans la solution analysée.

Dans cet exemple, l'essai (c'est-à-dire l'application du gel puis le séchage du gel, puis la re-dispersion) ayant été effectué dans le noir, l'ensemble du Cr est nécessairement sous la forme de Cr(VI). Le chrome relargué par les paillettes est donc présent dans le lixiviat, et la teneur en Cr(VI) dans le lixiviat ([Cr(VI)] (lixiviat)) est alors comparée à la teneur en Cr(VI) initiale ([Cr(VI)]$_0$) utilisée pour la contamination.

Comme il a été montré que les paillettes contiennent initialement l'ensemble de la contamination dans ce cas (voir exemple 2), ces mesures permettent de connaître la quantité de chrome toujours présente dans les paillettes après lixiviation. Les résultats obtenus sont donnés dans le Tableau 2 ci-dessous.

Tableau 2 : Résultats obtenus suite à la lixiviation des paillettes de gel à base de TiO$_2$.

| Absorbance à 352 nm | [Cr(VI)] (lixiviat) | [Cr(VI)] (lixiviat) / [Cr(VI)]$_0$ |
|---|---|---|
| 0,147 | 8,76.10$^{-5}$M | 0,350 |

Ces résultats montrent que, même après 2h de lixiviation des paillettes issues du gel à base de TiO$_2$, dans H$_2$O, sous agitation mécanique, seulement 35% du chrome est relargué en solution.

Le même essai de décontamination que celui réalisé avec le gel à base de TiO$_2$, est ensuite réalisé avec le gel comparatif à base de SiO$_2$ en suivant le même protocole, toujours dans le noir.

Après séchage, re-dispersion des paillettes, centrifugation et filtration, on analyse le lixiviat par spectroscopie UV-Visible.

Le spectre obtenu pour le lixiviat obtenu à partir des paillettes du gel à base de SiO$_2$ est donné sur la Figure 2.

L'utilisation de la loi de Beer-Lambert permet finalement de déterminer la concentration en Cr(VI) présent dans le lixiviat, et par conséquent la quantité de Cr(VI) toujours présente dans les paillettes à base de silice. Les résultats obtenus sont donnés dans le Tableau 3 ci-dessous.

Tableau 3 : Résultats obtenus suite la lixiviation des paillettes du gel à base de $SiO_2$.

| Absorbance à 352 nm | [Cr(VI)] (lixiviat) | [Cr(VI)] (lixiviat) / [Cr(Vbl)]$_0$ |
|---|---|---|
| 0,382 | $2,27.10^{-4}$M | 0,91 |

Ces résultats montrent que, même après 2h de lixiviation des paillettes à base de silice, dans $H_2O$, sous agitation mécanique, 91%, soit la quasi-totalité du chrome, est relarguée en solution.

Au vu de cet exemple, la comparaison de la capacité du gel à base de $TiO_2$, et de la capacité du gel comparatif à base de $SiO_2$, à retenir le chrome lors de la remise en solution des paillettes, démontre bien le pouvoir confinant significatif du gel à base de $TiO_2$. Le pouvoir confinant du gel à base de $TiO_2$ est bien plus élevé que celui du gel comparatif à base de $SiO_2$. Donc, l'aptitude du gel à base de $TiO_2$ à limiter un relargage de la pollution en cas d'altération des paillettes après traitement des surfaces contaminées, est excellente, et meilleure que celle du gel comparatif à base de $SiO_2$.

Exemple 4 non conforme à l'invention:

Dans cet exemple, on montre l'efficacité de la décontamination d'une surface poreuse à l'aide d'un gel à base de $TiO_2$.

A titre de comparaison, on effectue le même essai de décontamination à l'aide d'un gel de décontamination « classique » à base de $SiO_2$.

Plus exactement, il s'agit dans cet exemple de démontrer le pouvoir adsorbant d'un gel à base de $TiO_2$, notamment sur un support poreux, à savoir, ici, un support en béton.

Le gel à base de $TiO_2$ testé est le gel acide (pH = 5), qui est décrit dans l'exemple 1) non conforme à l'invention, il est comparé à un gel à base de $SiO_2$ qui est celui décrit dans l'exemple 3.

Au début, le protocole opératoire de l'essai de décontamination est le même que celui observé dans l'exemple 2 non conforme à l'invention. Seul le support change, en effet on utilise du béton en lieu et place du carreau de céramique.

Après séchage et récupération des paillettes de chacun des gels, celles-ci sont dissoutes sous agitation mécanique dans de l'acide fluorhydrique pur à 80°C pendant plusieurs heures et les solutions sont analysées par spectrométrie à émission optique à plasma induit (« *Inductively Coupled Plasma - Optical Emission Spectroscopy* » ou « *ICP-OES* » en anglais) avec un appareil ThermoFisher Scientific iCAP 6000 Sériés®, afin de déterminer les concentrations en chrome au sein de ces paillettes.

Les résultats obtenus sont donnés dans le Tableau 4 ci-dessous.

On note ici que la masse initiale de chrome hexavalent déposée sur chacun des supports est de 0,26 mg.

Tableau 4 : Résultats ICP-OES obtenus suite à la dissolution des paillettes des gels obtenues dans l'exemple 4.

| Gel | Masse des paillettes | Volume total de HF | Masse de Chrome déterminé par ICP | % de décontamination |
|---|---|---|---|---|
| A base de $TiO_2$ | 364,8 mg | 55 mL | 0,1 mg | 38% |
| A base de $SiO_2$ | 342,6 mg | 45 mL | < l.d. | 0% |
| < l.d. = Inférieur à la limite de détection de l'appareil. | | | | |

Ces résultats démontrent ainsi le pouvoir adsorbant du gel à base de $TiO_2$. En effet, sur un support poreux tel que le béton, le gel à base de $TiO_2$ permet de décontaminer au moins une partie du chrome présent dans les pores du béton, au contraire du gel de décontamination « classique », comparatif, non conforme à l'invention, à base de $SiO_2$.

Exemple 5 :

Dans cet exemple, on démontre l'effet photo-catalytique du gel selon l'invention, induisant la réduction du Cr(VI) et donc la diminution de sa toxicité.

Plus exactement, il s'agit dans cet exemple de démontrer l'influence d'un rayonnement UV ou visible sur la réduction de la toxicité du Cr(VI), après la décontamination d'une surface contaminée par du Cr(VI), par application sur cette surface d'un gel à base de $TiO_2$ selon l'invention.

Pour cet exemple, et l'exemple 7 qui suit, (en effet, il n'y a pas de mesure de la quantité de Cr dans l'exemple 6, seulement une observation visuelle), la mesure de la quantité de chrome hexavalent présent dans le gel se fera après lixiviation des paillettes de gel sec par de l'eau, suivie d'une analyse UV-Visible selon le protocole présenté dans l'exemple 3.

On considère que le rapport $[Cr(VI)](lixiviat)/[Cr(VI)]_0$ est représentatif de la réduction (au sens de réduction chimique) du chrome hexavalent total.

En effet, bien que la mesure ne soit effectuée que sur environ un tiers du chrome total présent dans les paillettes (seulement 35% du chrome présent dans les paillettes passe en solution après lixiviation à l'eau), on admet que les atomes de chrome adsorbés en surface des particules de $TiO_2$, et toujours présents au sein des paillettes, ont au moins autant de chance d'avoir été réduits que ceux présents dans le lixiviat.

Le gel selon l'invention utilisé dans cet exemple possède la composition suivante :

Tableau 5 : Composition du gel utilisé dans l'exemple 5.

| Gel | % massique de $TiO_2$ | % massique de $H_2O$ | % massique d'EtOH | pH (initial) | pH (gel) |
|---|---|---|---|---|---|
| Gel $H_2O$-40 EtOH | 20 | 40 | 40 | 3,7 | 5 |

Ce gel conforme à l'invention est préparé de la manière suivante :
Dans un premier temps, les particules de $TiO_2$ sont dispersées dans le solvant (eau et éthanol) à l'aide d'un agitateur mécanique, muni d'un agitateur à trois pales, à une vitesse de 200 tours/min.

On obtient ainsi une suspension liquide blanche opaque dont le pH est d'environ 3,5.

On augmente alors progressivement le pH, toujours sous agitation, à l'aide de soude NaOH 0,1 M, jusqu'à ce que le pH soit égal à 5, ce qui va permettre de former un gel visqueux blanc opaque.

Pendant l'addition de soude, on augmente graduellement l'agitation, au fur et à mesure que la viscosité croît, pour arriver à environ 400 à 600 tours/min sans qu'il n'y ait de projections. Le gel est ensuite maintenu sous agitation pendant 5 minutes.

On note ici que la quantité de soude ajoutée est systématiquement négligeable par rapport à la quantité des solvants, on peut ainsi considérer que la composition massique de 20% de $TiO_2$ de 40% de $H_2O$, et de 40% d'éthanol, reste constante une fois à pH = 5.

Le pH du gel ainsi préparé est donc faiblement acide car égal à 5.

L'efficacité réductrice des gels de cet exemple est suivie par analyse par spectrométrie UV-Visible, à partir de laquelle on détermine la concentration en Cr(VI).

Dans un premier temps, 0,25mL d'une solution de $K_2Cr_2O_7$ à $10^{-2}$ M (de chez Sigma-Aldrich ® avec une pureté $\geq$ 99.0%, dissous dans $H_2O$), équivalente à une solution concentrée à $2.10^{-2}$M en Cr(VI), sont déposés sur un carreau de céramique.

Cette opération est réalisée sur deux carreaux de céramique distincts.

On laisse ensuite sécher les gouttes pendant toute une nuit, moyennant quoi on obtient une tâche sur chacun des deux carreaux.

Le gel décrit plus haut dans cet exemple est déposé sur la tâche d'un premier carreau de céramique puis on place le tout sous le rayonnement d'une lampe UV (lampe UV de chez Vilber®, $\lambda$ = 365 nm) avec une épaisseur de 1,25 mm.

Le gel décrit plus haut est également déposé avec une épaisseur de 1,25 mm sur la tâche du deuxième carreau de céramique et le tout est laissé sous lumière visible.

Puis, on laisse sécher le premier échantillon (constitué par une tâche jaune sur laquelle est déposé le gel) pendant trois heures sous le rayonnement d'une lampe UV (lampe UV de chez Vilber®, $\lambda$ = 365 nm), et le second échantillon en plein jour, sous lumière visible.

Les paillettes de gel sec finalement obtenues sont récupérées par brossage.

Concernant le gel séché sous UV, les paillettes apparaissent légèrement vertes (qui est la couleur caractéristique de la présence de Cr(III)) ou marrons, alors que les paillettes du gel séché en plein jour, sous lumière visible, possèdent encore des teintes jaunes.

Dans un second temps, les paillettes de gel sec sont re-dispersées dans 20 mL d'$H_2O$ pendant une heure.

Après centrifugation et filtration, le lixiviat est analysé par spectroscopie UV-Visible.

Les spectres obtenus sont présentés sur la Figure 3 (à titre de comparaison, la courbe représentative du gel à base de $TiO_2$ de l'exemple 1, dont le solvant est uniquement de l'eau et qui est séché dans le noir a été ajoutée).

Les résultats obtenus par application de la loi de Beer-Lambert sont résumés dans le Tableau 6 ci-dessous.

D'une part, l'analyse du chrome dans le lixiviat, montre que le chrome dans le lixiviat correspond à environ 35% du chrome total contenu dans les paillettes, comme cela a été montré dans l'exemple 3 non conforme à l'invention.

D'autre part, les paillettes contiennent l'ensemble du chrome initial car il a été montré que le gel permettait l'extraction totale du chrome présent sur le substrat lisse.

Ainsi la proportion en Cr(VI) dans les paillettes peut être estimée par l'équation suivante :

$$\% \; en \; Cr(VI)_{dans \; les \; paillettes} = 100 \times \frac{[Cr(VI)]_{analysé \; dans \; les \; paillettes}}{0.35[Cr(VI)]_0}$$

Tableau 6 : Résultats obtenus suite la lixiviation des paillettes de gel obtenues dans l'exemple 5.

| Rayonnement | Absorbance | [Cr(VI)] (lixiviat) | % Cr(VI) dans les paillettes de gel |
|---|---|---|---|
| UV | 0,001 | 0 | 0 |
| Visible | 0,032 | $1,9.10^{-5}$ M | 21,7% |

Ces résultats montrent que l'utilisation d'un rayonnement UV permet d'obtenir une réduction totale du chrome hexavalent (quasiment 100%) alors que pour le gel laissé à sécher en plein jour, sous lumière visible, seulement 78,3% de réduction du chrome hexavalent sont obtenus.

Cet exemple met cependant en évidence le fait que le rayonnement visible permet d'activer, au moins en partie, les particules de $TiO_2$, car 78,3% de réduction du Cr(VI) sont obtenus.

Exemple 6 non conforme à l'invention:

Dans cet exemple, on montre l'effet photo-catalytique d'un gel à base de $TiO_2$, sur la destruction d'un polluant chimique.

Plus exactement, il s'agit dans cet exemple de démontrer le pouvoir photocatalytique d'un gel à base de $TiO_2$, sur la dégradation de composés organiques.

A titre de composé organique à détruire, dégrader, on utilise un colorant organique, le Rouge de Méthyle (Sigma-Aldrich®) sous la forme d'une solution de ce colorant à 40 mg/L.

Ce colorant organique est rouge pour des pH inférieurs à 4,4, orange pour des pH entre 4,4 et 6,2, et jaune pour des pH supérieurs à 6,2.

4 gouttes de la solution de ce colorant sont déposées sur un support lisse, à savoir un carreau de céramique. Les gouttes sont laissées à sécher pour obtenir ainsi 4 tâches, et trois gels différents sont déposés à la spatule sur trois de ces tâches, tandis qu'une dernière tâche reste nue, ne reçoit pas de gel, et joue donc le rôle de tâche de colorant témoin.

Les 3 gels sont :

- un gel à base de $TiO_2$ acide (pH = 5), tel que décrit dans l'exemple non conforme à l'invention.
- un gel comparatif à base de $SiO_2$ tel que décrit dans l'exemple 3. Ce gel est un gel acide (pH = 4,5).
- un gel comparatif à base d'Abus. Ce gel est un gel légèrement basique (pH = 8).

Le gel comparatif, à base de $Al_2O_3$, est un gel dont la composition est la suivante en pourcentages massiques :

- 17% d'alumine;
- 83% d'eau distillée.

L'alumine est l'alumine commercialisée par la société EVONIK® sous la dénomination Aeroxide® Alu C.

Ce gel comparatif, à base d'$Al_2O_3$ est préparé de la manière suivante :
L'alumine est ajoutée progressivement dans de l'eau distillée, sous agitation mécanique, à l'aide d'un agitateur mécanique muni d'un agitateur à trois pales à une vitesse de 200 tours/min. Pendant l'addition d'alumine, on augmente graduellement l'agitation, au fur et à mesure que la viscosité croit, pour arriver à environ 400 à 600 tours/min sans qu'il n'y ait de projections. Le gel est ensuite maintenu sous agitation pendant 5 minutes.

Le pH de ce gel est mesuré à 8.

On laisse ensuite sécher ces gels déposés sous rayonnement UV (lampe UV de chez Vilber, $\lambda$ = 365 nm) pendant 4h30.

Après séchage sous UV, la tâche de colorant témoin est intacte, les paillettes du gel comparatif à base d'$Al_2O_3$, légèrement basique, sont jaunes, ce qui est en accord avec le caractère basique de ce gel, et les paillettes du gel comparatif à base de $SiO_2$, acide, sont rouges, ce qui est en accord avec le caractère acide de ce gel. Le colorant organique n'a donc pas été dégradé par ces gels comparatifs.

Par contre, les paillettes du gel à base de $TiO_2$ sont totalement blanches, ce qui prouve la dégradation du colorant organique dû au pouvoir photocatalytique du $TiO_2$ sous rayonnement UV.

On démontre ainsi dans cet exemple la possibilité de dégrader des composés organiques (tels que les contaminants chimiques ou biologiques) présents en surface d'un matériau grâce à l'utilisation d'un gel photo-activable à base de $TiO_2$.

Exemple 7 :

Dans cet exemple, on étudie l'influence de la présence d'éthanol dans des gels à base de $TiO_2$ selon l'invention.

Trois compositions de gel, dont deux sont conformes à l'invention, sont étudiées dans cet exemple.

Ces gels sont préparés de la même manière que dans l'exemple 1, si ce n'est que pour les deux derniers gels préparés, qui sont les gels conformes à l'invention, le solvant est modifié et comprend un mélange d'eau et d'éthanol, et non plus seulement de l'eau (voir tableau 7 ci-dessous).

Une nouvelle fois, on peut considérer que les quantités massiques de soude ajoutées sont négligeables par rapport aux masses du ou des solvants, on peut ainsi considérer que les compositions massiques restent constantes une fois à pH = 5.

Tableau 7 : Composition des gels étudiés dans l'exemple 7.

| Gel | % massique de $TiO_2$ | % massique de $H_2O$ | % massique d'EtOH | pH (initial) | pH (gel) |
|---|---|---|---|---|---|
| Gel $H_2O$ | 20 | 80 | 0 | 3,5 | 5 |
| Gel $H_2O$-24 EtOH | 20 | 56 | 24 | 3,7 | 5 |
| Gel $H_2O$-40 EtOH | 20 | 40 | 40 | 3,7 | 5 |

L'efficacité réductrice des gels de cet exemple est démontrée en utilisant le même protocole de mesure que celui présenté dans l'exemple 5, mais seul le séchage sous lampe UV est utilisé.

Après séchage, re-dispersion des paillettes, centrifugation et filtration, on analyse le lixiviat par spectroscopie UV-Visible. Pour chacun des gels étudiés, les paillettes séchées sont de couleur verte (couleur caractéristique de la présence de Cr(III)) ou marron, et plus aucune trace jaune n'est observée sur le support.

Les spectres UV-visible sont présentés sur la La Figure 4 (à titre de comparaison, la courbe représentative du gel à base de $TiO_2$ de l'exemple 1 non conforme à l'invention, dont le solvant est uniquement de l'eau et qui est séché dans le noir a été ajoutée).

Les résultats obtenus par application de la loi de Beer-Lambert sont donnés dans le Tableau 8 ci-dessous.

Le % de Cr(VI) dans les paillettes est calculé de la même façon que dans l'exemple 5.

Tableau 8 : Résultats obtenus suite la lixiviation des paillettes de gel obtenues dans l'exemple 7.

| Gel | Absorbance à 352 nm | [Cr(VI)] (lixiviat) | % Cr(VI) dans les paillettes de gel |
|---|---|---|---|
| **Gel** $H_2O$ | 0,006 | $3,6.10^{-6}$M | 4,1% |
| Gel $H_2O$-24 EtOH | 0 | 0 | 0 |
| Gel $H_2O$-40 EtOH | 0 | 0 | 0 |

Ces résultats montrent que, pour les trois compositions de gel présentées dans cet exemple, avec un séchage sous UV, on obtient d'excellents résultats, car au moins 95,9% du chrome hexavalent est réduit.

On note que la présence d'éthanol dans les gels selon l'invention, agissant comme un élément sacrificiel pour éviter la recombinaison électron-trou, permet une légère amélioration de l'efficacité réductrice du gel pour arriver à 100% de chrome hexavalent réduit.

Exemple 8 :

Dans cet exemple, on montre que les gels selon l'invention peuvent être mis en œuvre par pulvérisation.

Une étude rhéologique sur deux des trois gels décrits dans l'exemple 7, à savoir le gel « $H_2O$ » et le gel « $H_2O$-

40 EtOH » selon l'invention a été réalisée, et a permis de montrer que ces gels sont adaptés à une mise en œuvre par pulvérisation.

Pour que ces gels puissent être appliqués par un procédé de pulvérisation, il faut qu'ils possèdent les propriétés d'un fluide rhéofluidifiant, thixotrope avec un temps de reprise très court (inférieur à la seconde) et avec une contrainte seuil (typiquement supérieure à 15-20 Pa).

Différentes mesures rhéologiques ont ainsi été réalisées à l'aide d'un rhéomètre TA Instruments® AR-1000 en géométrie Vane, et sont présentées dans cet exemple.

Dans un premier temps, la viscosité du gel a été mesurée en fonction du taux de cisaillement. Après un pré-cisaillement de 5 minutes à un taux de cisaillement de 20 $s^{-1}$ puis de 1 minute à $6,72.10^{-3}$ $s^{-1}$, plusieurs paliers de taux de cisaillement allant de $6,2.10^{-3}$ $s^{-1}$ à 100 $s^{-1}$ sont effectués avec une mesure de la viscosité toutes les 30 secondes.

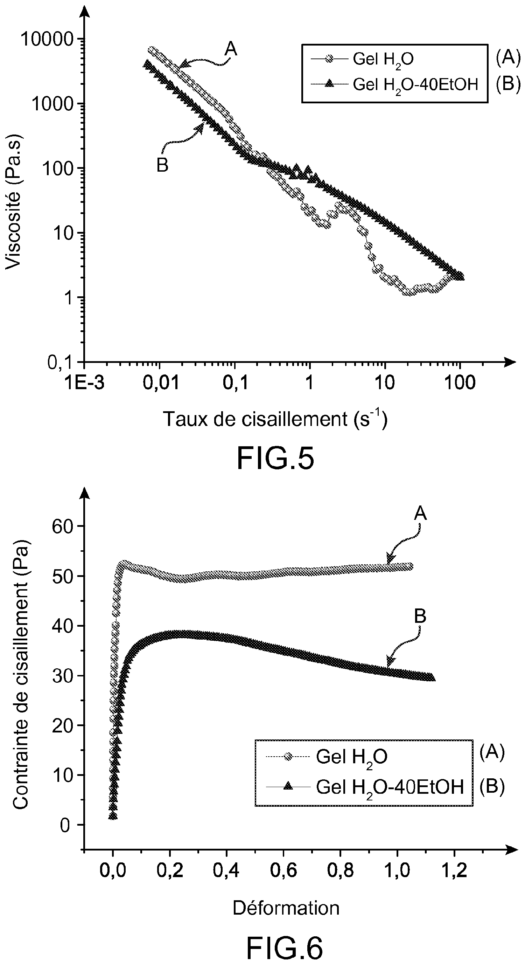

La Figure 5 donne l'évolution de la viscosité (Pa.s) des deux gels étudiés dans cet exemple en fonction du taux de cisaillement $s^{-1}$) pour des taux de cisaillement compris entre $6.72.10^{-3}$ et 100 $s^{-1}$.

On observe, pour chacun des gels, une chute drastique de la viscosité avec le taux de cisaillement, caractéristique d'un comportement rhéofluidifiant.

On constate de plus, que la présence d'éthanol dans le gel conforme à l'invention, tend à rendre le comportement rhéofluidifiant du gel plus parfait. En effet, la valeur de viscosité du gel « $H_2O$-40 EtOH » selon l'invention en fonction du taux de cisaillement évolue de manière plus linéaire que celle du gel « $H_2O$ » qui, elle, présente des sauts de manière assez irrégulière.

D'autre part, les valeurs de contrainte seuil de ces deux gels décrits dans l'exemple 7 (gel « $H_2O$ » et gel « $H_2O$-40 EtOH » selon l'invention) sont déterminées en mesurant l'évolution de leur contrainte de cisaillement ainsi que leur déformation à taux de cisaillement imposé.

Un faible taux de cisaillement ($6,72.10^{-3}$ $s^{-1}$) est alors appliqué à chacun des gels de manière constante afin de les déformer en partant du repos et ainsi de déterminer leur seuil d'écoulement.

La Figure 6 représente la contrainte de cisaillement en fonction de la déformation obtenue pour les deux gels décrits dans l'exemple 7.

Les deux courbes possèdent la même allure : deux régimes sont observés. Tout d'abord la contrainte augmente fortement, le matériau est en régime solide (déformation élastique). On observe ensuite un changement de comportement, la contrainte atteint le seuil d'écoulement et le matériau passe en régime liquide (écoulement stationnaire). La contrainte seuil correspond alors à la contrainte au seuil d'écoulement du gel, soit 52 Pa pour le Gel « $H_2O$ » et 36 Pa pour le Gel « $H_2O$-40 EtOH ». Cette contrainte seuil est donc bien supérieure à 20 Pa ce qui va permettre au gel de tenir sur une paroi pour des épaisseurs comprises entre 0 et au moins 2 mm.

Pour conclure sur cet exemple, les gels $H_2O$ et $H_2O$-40EtOH possèdent bien les propriétés rhéologiques adéquates permettant d'être très facilement pulvérisables sur différents types de surfaces (horizontales ou non). La présence d'éthanol dans le gel selon l'invention influe significativement sur la rhéologie du gel : elle permet d'une part de tendre vers un caractère rhéofluidifiant plus marqué mais, d'autre part, le gel possède une contrainte seuil légèrement plus basse et s'écoulera donc plus facilement.

Exemple 9 :

Dans cet exemple, on montre que les gels selon l'invention peuvent être mis en œuvre par pulvérisation.

Pour que les gels selon l'invention puissent être appliqués par une technique de pulvérisation, il faut qu'ils possèdent les propriétés d'un fluide rhéofluidifiant.

Deux compositions de gel conformes à l'invention sont étudiées dans cet exemple.

Ces gels sont préparés de la même manière que dans l'exemple 1 non conforme à l'invention.

Une nouvelle fois, on peut considérer que les quantités massiques de soude ajoutées sont négligeables par rapport aux masses de solvant.

Tableau 9 : Compositions des gels étudiés dans l'exemple 9.

| Gel | % massique de $TiO_2$ | % massique de $H_2O$ | % massique d'EtOH | pH (initial) | pH (gel) |
|---|---|---|---|---|---|
| Til5_pH 5,5 | 15 | 42,5 | 42,5 | 3,7 | 5,5 |
| Til5_pH 9 | 15 | 42,5 | 42,5 | 3,7 | 9 |

Des mesures rhéologiques ont ainsi été réalisées à l'aide d'un rhéomètre TA Instruments® AR-1000 en géométrie Vane, et sont présentées dans cet exemple.

La viscosité du gel a été mesurée en fonction du taux de cisaillement.

Après un pré-cisaillement de 5 minutes à un taux de cisaillement de 0.01 $s^{-1}$, plusieurs paliers de taux de cisaillement allant de 0.01 s$^{-1}$ à 100 s$^{-1}$ sont effectués et la viscosité est mesurée.

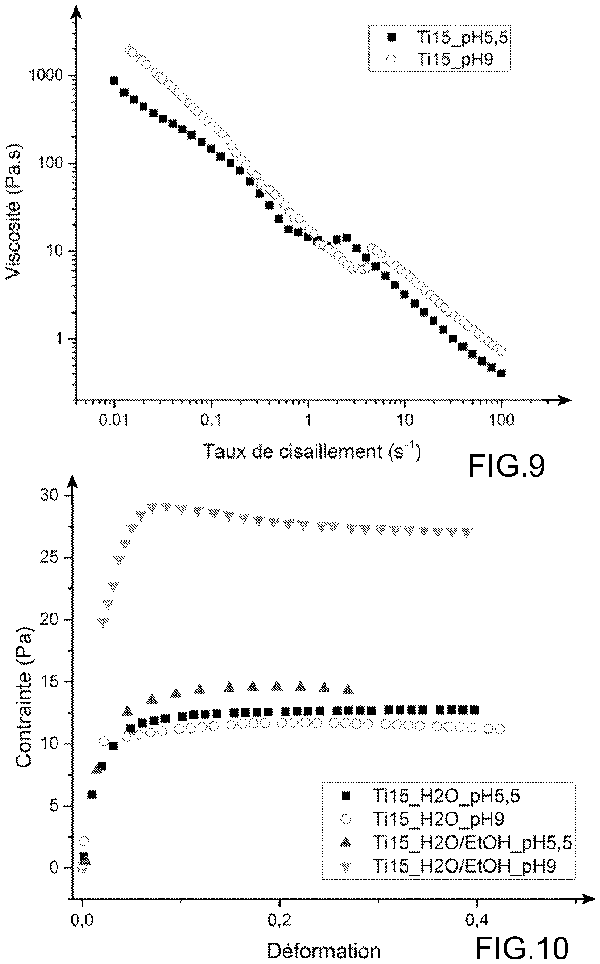

La Figure 9 donne l'évolution de la viscosité (Pa.s) des deux gels étudiés dans cet exemple 9 en fonction du taux de cisaillement (s$^{-1}$) pour des taux de cisaillement compris entre 0.01 et 100 s$^{-1}$.

On observe pour chacun des gels, une chute drastique de la viscosité avec le taux de cisaillement, caractéristique d'un comportement rhéofluidifiant.

En conclusion de cet exemple, on peut dire que les deux gels de cet exemple Til5_pH 5,5 et Til5_pH 9 sont bien rhéofluidifiants, et peuvent donc être mis en œuvre par pulvérisation.

Exemple 10 :

Dans cet exemple, on montre que les gels selon l'invention peuvent tenir sur une épaisseur de plusieurs millimètres sur des parois non-horizontales telles qu'un mur ou un plafond.

Pour cela, il faut que les gels étudiés possèdent un seuil d'écoulement suffisamment important, c'est-à-dire une contrainte seuil supérieure à 10 Pa.

Quatre compositions de gel sont étudiées dans cet exemple. Ces gels sont préparés de la même manière que dans l'exemple 1.

Parmi ces quatre compositions de gel, deux sont conformes à l'invention, à savoir les compositions de gel dénommées « Ti15_H$_2$O/EtOH_pH 5,5 », et « Ti15_ H$_2$O/EtOH_pH 9 ».

Une nouvelle fois, on peut considérer que les quantités massiques de soude ajoutées sont négligeables par rapport aux masses du solvant.

Tableau 10 : Compositions des gels étudiés dans l'exemple 10.

| Gel | % massique de TiO$_2$ | % massique de H$_2$O | % massique d'EtOH | pH (initial) | pH (gel) |
|---|---|---|---|---|---|
| Ti15_H$_2$O_pH 5,5 | 15 | 85 | 0 | 3,7 | 5,5 |
| Ti15_ H$_2$O_pH 9 | 15 | 85 | 0 | 3,7 | 9 |
| Ti15_H$_2$O/EtOH_pH 5,5 | 15 | 42,5 | 42,5 | 3,7 | 5,5 |
| Ti15_ H$_2$O/EtOH_pH 9 | 15 | 42,5 | 42,5 | 3,7 | 9 |

Les valeurs de contrainte seuil des gels décrits dans cet exemple sont déterminées en mesurant l'évolution de leur contrainte de cisaillement ainsi que leur déformation à taux de cisaillement imposé.

Des mesures ont été réalisées à l'aide d'un rhéomètre TA Instruments® AR-1000 en géométrie Vane et sont présentées dans cet exemple.

Un faible taux de cisaillement (0.01 s$^{-1}$) est appliqué à chacun des gels de manière constante afin de les déformer en partant du repos et ainsi de déterminer leur seuil d'écoulement.

La Figure 10 représente la contrainte de cisaillement en fonction de la déformation obtenue pour les quatre gels décrits dans cet exemple.

Les quatre courbes possèdent la même allure : deux régimes sont observés. Tout d'abord la contrainte augmente fortement, le matériau est en régime solide (déformation élastique).

On observe ensuite un changement de comportement, la contrainte atteint le seuil d'écoulement et le matériau passe en régime liquide (écoulement stationnaire). La contrainte seuil correspond alors à la contrainte au seuil d'écoulement du gel, soit 12 Pa pour le Gel « Ti15_H$_2$O_pH 5,5 », 11 Pa pour le Gel « Ti15_H$_2$O_pH 9 », 14 Pa pour le Gel « Ti15_H$_2$O/EtOH_pH 5.5 » et 29 Pa pour le Gel « Ti15_H$_2$O/EtOH_pH 9 ». Ces contraintes seuil sont donc bien supérieures à 10 Pa ce qui va permettre aux gels de tenir sur une paroi pour des épaisseurs comprises entre 0 et au moins 1 mm.

On observe de plus que la présence d'éthanol dans les gels selon l'invention permet d'augmenter le seuil d'écoulement du gel et donc de pouvoir déposer une épaisseur de gel plus importante sur des parois non-horizontales telles que des murs ou des plafonds.

Exemple 11 :

Dans cet exemple, on montre que, après application et séchage, les gels selon l'invention sont aspirables.

Plus exactement, il s'agit dans cet exemple de montrer que les gels selon l'invention, décrits dans les exemples précédents, sèchent bien dans un temps raisonnable de quelques heures, et qu'ils se fracturent en produisant des paillettes non pulvérulentes de taille millimétrique pouvant être facilement aspirées.

L'influence de la présence d'éthanol est également montrée dans cet exemple, en particulier en ce qui concerne le temps de séchage.

Pour réaliser cette étude, le gel « $H_2O$ », et le gel « $H_2O$-24 EtOH » selon l'invention sont un à un mis à sécher dans une enceinte climatique Binder® dont la température et le pourcentage d'humidité relative sont fixés à 25°C et 50% respectivement.

Les gels sont étalés sur une nacelle en acier inoxydable usinée de façon à obtenir une épaisseur contrôlée de 2 mm de gel dans la nacelle.

Dans l'enceinte climatique, une balance de précision Sartorius® est installée, de même qu'une caméra Moticam® entourée d'une lampe à LED circulaire (VWR) qui est placée sur le dessus de la balance.

La balance et la caméra Moticam® sont reliées à un ordinateur placé en-dehors de l'enceinte climatique permettant ainsi l'acquisition simultanée, au cours du séchage en atmosphère contrôlée, de la masse et des images de la nacelle remplie de gel.

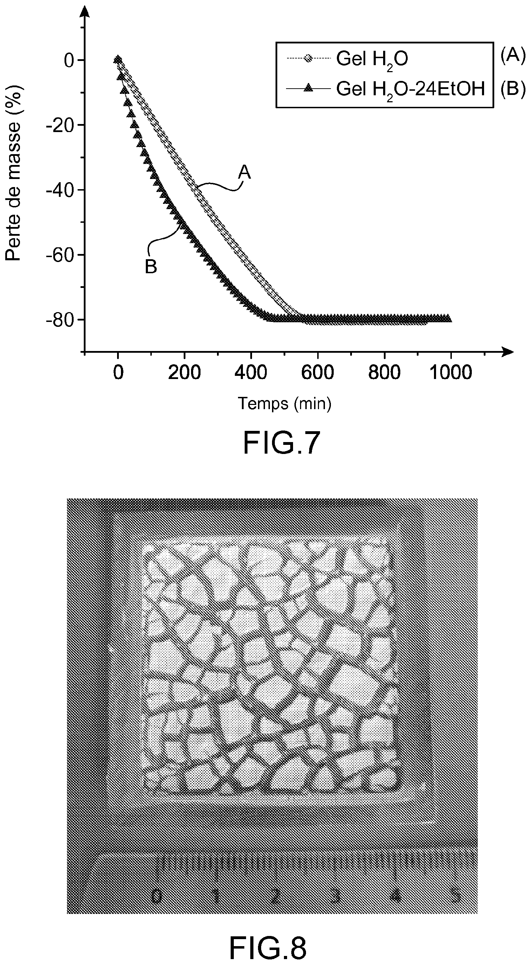

Il est à noter que la nacelle contenant le gel est placée dans la balance de précision et que toutes les portes de la balance sont fermées, exception faite de la porte opposée à la soufflerie qui est ouverte de 3 cm (pour maintenir l'atmosphère contrôlée dans l'habitacle de la balance tout en limitant le flux d'air lié au fonctionnement de l'enceinte climatique). L'enregistrement de la masse au cours du séchage permet alors de tracer une cinétique de séchage. L'ensemble des résultats est porté sur la Figure 7.

On observe ainsi que les deux gels étudiés dans cet exemple sèchent bien, en une durée maximale de seulement quelques heures, à savoir 580 minutes, soit 9 heures et 40 minutes, pour le gel « $H_2O$ », et 480 minutes, soit 8 heures pour le gel « $H_2O$-24 EtOH » conforme à l'invention.

La présence d'éthanol dans la formulation du gel selon l'invention permet ainsi de diminuer le temps de séchage du gel car l'évaporation de l'éthanol s'effectue à une température plus basse que l'évaporation de l'eau.

Concernant la fracturation, une photographie des paillettes sèches obtenues pour un gel $H_2O$-24 EtOH selon l'invention déposé dans une nacelle de 2 mm de profondeur est donnée sur la Figure 8.

Il apparaît que le nombre de paillettes formées et surtout leur taille est bien conforme au fait que ces paillettes sont de tailles millimétriques et ne sont pas pulvérulentes.

## REFERENCES

[1] Faure, S., et *al., "Procédé de traitement d'une surface par un gel de traitement, et gel de traitement",* 2003, FR-A1-2 827 530.

[2] Faure, S., P. Fuentes, et Y. Lallot, *"Gel aspirable pour la décontamination de surfaces et utilisation",* 2007, FR-A1-2 891470.

[3] Cuer, F. et S. Faure, *"Gel de décontamination biologique et procédé de décontamination de surfaces utilisant ce gel",* 2010, WO-A1-2012001046.

[4] Ludwig, A., F. Goettmann, et F. Frances, *"Gel alcalin oxydant de décontamination biologique et procédé de décontamination biologique de surfaces utilisant ce gel",* 2013, WO-A1-2014154818.

[5] DE 10 2007 014 875 A1 (HENKEL AG) 2 octobre 2008

## Revendications

**1.** Gel de décontamination adsorbant et photocatalytique, constitué par une solution colloïdale comprenant, de préférence constituée par :

- 15% à 20% en masse, mieux 15% à 20% en masse la valeur 15% étant exclue, mieux encore 16% à 20% en masse, par exemple 20% en masse de $TiO_2$, éventuellement dopé, par rapport à la masse du gel;
- éventuellement, 0,01% à 10% en masse, de préférence 0,1% à 5% en masse, par rapport à la masse du gel, d'au moins un colorant et/ou d'au moins un pigment ;
- éventuellement, 0,1% à 2% en masse, par rapport à la masse du gel, d'au moins un agent tensio-actif ;
- éventuellement, 0,05% à 5% en masse, de préférence 0,05% à 2% en masse, par rapport à la masse du gel, d'au moins un polymère super-absorbant ;
- et le reste de solvant, ledit solvant étant choisi parmi les mélanges d'eau dans une proportion de 40% à 56 % en masse, par exemple 40%, 42,5%, ou 56% en masse, et d'éthanol, dans une proportion de 24% à 42,5% en masse, de préférence de 24% à 40% en masse, par exemple 24 %, 40 %, ou 42,5 % en masse par rapport à la masse du gel ; et ledit gel ayant un pH supérieur ou égal à 4.

2. Gel selon la revendication 1, qui a un pH faiblement acide de 4 à moins de 7; ou bien un pH neutre de 7 ; ou bien un pH faiblement basique de plus de 7 à moins de 9; ou bien un pH très basique de 9 ou plus.

3. Gel selon la revendication 1 ou 2, dans lequel le pH du gel est ajusté par addition d'une base minérale, choisie par exemple parmi la soude NaOH, la potasse KOH, et leurs mélanges.

4. Gel selon l'une quelconque des revendications précédentes, dans lequel le $TiO_2$ se présente sous la forme de particules d'une taille moyenne de 2 à 200 nm.

5. Procédé de décontamination d'au moins une surface d'un substrat en un matériau solide, ladite surface étant contaminée par au moins une espèce contaminante se trouvant sur ladite surface et éventuellement sous ladite surface (en subsurface) dans la profondeur du substrat, dans lequel on réalise au moins un cycle comprenant les étapes successives suivantes :

a) on applique le gel selon l'une quelconque des revendications 1 à 4 sur ladite surface, par exemple par pulvérisation, au pinceau ou avec une taloche ;
b) on maintient le gel sur la surface au moins pendant une durée suffisante pour que le gel absorbe l'espèce contaminante, puis pour que l'espèce contaminante soit adsorbée à la surface des particules de $TiO_2$, et pour que le gel sèche et forme un résidu sec et solide contenant ladite espèce contaminante adsorbée à la surface des particules de $TiO_2$ ;
c) on élimine le résidu sec et solide contenant ladite espèce contaminante adsorbée dans le gel à la surface des particules de $TiO_2$, par exemple le résidu sec et solide est éliminé de la surface solide par brossage et/ou aspiration.

6. Procédé selon la revendication 5, dans lequel le substrat est en au moins un matériau solide choisi parmi les métaux et les alliages métalliques comme l'acier inoxydable, les aciers peints, l'aluminium, et le plomb ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et matériaux cimentaires ; les mortiers et bétons ; les plâtres ; les briques ; la pierre naturelle ou artificielle ; les céramiques.

7. Procédé selon l'une quelconque des revendications 5 et 6, dans lequel l'espèce contaminante est choisie parmi les espèces contaminantes ioniques, chimiques, biologiques, nucléaires ou radioactives.

8. Procédé selon la revendication 7, dans lequel l'espèce contaminante est une espèce contaminante ionique choisie parmi les ions métalliques monovalents et multivalents, notamment parmi les ions métalliques monovalents et multivalents toxiques comme les ions chrome (VI), nickel (II), argent (I), cadmium (II), mercure (II), arsenic (III), et plomb (II) ; ou l'espèce contaminante est une espèce contaminante biologique, choisie parmi les bactéries, les champignons, les levures, les virus, les toxines, les spores notamment les spores de *Bacillus anthracis,* les prions, et les protozoaires, de préférence l'espèce contaminante biologique est choisie parmi les espèces biotoxiques telles que les spores pathogènes comme par exemple les spores de *Bacillus anthracis,* les toxines comme par exemple la toxine botulique ou la ricine, les bactéries comme les bactéries *Yersinia pestis* et les virus comme le virus de la vaccine ou les virus des fièvres hémorragiques par exemple de type Ebola ; ou l'espèce contaminante est choisie parmi les espèces chimiques toxiques, telles que les gaz toxiques, en particulier neurotoxiques ou vésicants, notamment les composés organophosphorés, comme le Sarin ou agent GB, le VX, le Tabun ou agent GA, le Soman, le Cyclosarin, le diisopropyl fluoro phosphonate (DFP), l'Amiton ou agent VG, le Parathion, le gaz moutarde ou agent H ou agent HD, la Lewisite ou agent L, l'agent T.

9. Procédé selon l'une quelconque des revendications 5 à 8, dans lequel le gel est appliqué sur la surface à décontaminer à raison de 100 g à 2000 g de gel par $m^2$ de surface, de préférence de 500 à 1500 g de gel par $m^2$ de surface, de préférence encore de 600 à 1000 g de gel par $m^2$ de surface, ce qui correspond généralement à une épaisseur de gel déposé sur la surface de 0,1 mm à 2 mm, de préférence de 0,5 mm à 2 mm, de préférence encore de 1 mm à 2 mm.

10. Procédé selon l'une quelconque des revendications 5 à 9, dans lequel, pendant tout ou partie de l'étape a), et/ou

**EP 3 484 610 B1**

pendant tout ou partie de l'étape b), de préférence pendant toute la durée de l'étape b), le gel maintenu sur la surface est exposé à un rayonnement visible ou à un rayonnement Ultraviolet A, B, ou C (UVA, UVB, ou UVC), ou à un autre rayonnement, de manière à inactiver, et/ou dégrader, et/ou réduire, et/ou détruire, l'espèce contaminante par photocatalyse.

11. Procédé selon l'une quelconque des revendications 5 à 10, dans lequel (lors de l'étape b)), le séchage est réalisé à une température de 1°C à 50°C, de préférence de 15°C à 25°C, et sous une humidité relative de 20% à 80%, de préférence de 20% à 70% ; le gel est maintenu sur la surface pendant une durée de 2 à 72 heures, de préférence de 2 à 48 heures, de préférence encore de 4 à 24 heures.

12. Procédé selon l'une quelconque des revendications 5 à 11, dans lequel le résidu sec et solide se présente sous la forme de particules, par exemple de paillettes, d'une taille de 1 à 10 mm, de préférence de 2 à 5 mm.

13. Procédé selon l'une quelconque des revendications 5 à 12, dans lequel le cycle est répété de 1 à 10 fois en utilisant le même gel lors de tous les cycles, ou en utilisant des gels différents lors d'un ou de plusieurs cycle(s).

14. Procédé selon l'une quelconque des revendications 5 à 13, dans lequel lors de l'étape b), le gel, avant séchage total, est remouillé avec un solvant, de préférence avec le solvant du gel appliqué lors de l'étape a).

**Patentansprüche**

1. Photokatalytisches, adsorbierendes Dekontaminationsgel, bestehend aus einer kolloidalen Lösung, enthaltend, vorzugsweise bestehend aus :

    - 15 bis 20 Masse-%, vorzugsweise 15 bis 20 Masse-%, wobei der Wert 15 % ausgeschlossen ist, noch bevorzugter 16 bis 20 Masse-%, beispielsweise 20 Masse-% $TiO_2$, gegebenenfalls dotiert, bezogen auf die Masse des Gels;
    - gegebenenfalls 0,01 bis 10 Masse-%, vorzugsweise 0,1 bis 5 Masse-%, bezogen auf die Masse des Gels, zumindest eines Farbstoffs und/oder zumindest eines Pigments;
    - gegebenenfalls 0,1 bis 2 Masse-%, bezogen auf die Masse des Gels, zumindest eines Tensids;
    - gegebenenfalls 0,05 bis 5 Masse-%, vorzugsweise 0,05 bis 2 Masse-%, bezogen auf die Masse des Gels, zumindest eines superabsorbierenden Polymers;
    - wobei der Rest Lösungsmittel ist und dieses Lösungsmittel ausgewählt ist aus Gemischen von Wasser in einem Anteil von 40 bis 56 Masse-%, beispielsweise 40%, 42,5% oder 56 Masse-%, und Ethanol in einem Anteil von 24 bis 42,5 Masse-%, vorzugsweise 24 bis 40 Masse-%, beispielsweise 24%, 40% oder 42,5 Masse-%, bezogen auf die Masse des Gels, wobei das Gel einen pH-Wert von größer oder gleich 4 aufweist.

2. Gel nach Anspruch 1, wobei es einen schwach sauren pH-Wert von 4 bis weniger als 7; oder einen neutralen pH-Wert von 7; oder einen schwach basischen pH-Wert von mehr als 7 bis weniger als 9; oder einen sehr basischen pH-Wert von 9 oder mehr aufweist.

3. Gel nach Anspruch 1 oder 2, wobei der pH-Wert des Gels durch Zugabe einer Mineralbase, beispielsweise ausgewählt aus Soda NaOH, Pottasche KOH und deren Gemischen, eingestellt wird.

4. Gel nach einem der vorangehenden Ansprüche, wobei das $TiO_2$ in Form von Teilchen mit einer durchschnittlichen Teilchengröße von 2 bis 200 nm vorliegt.

5. Verfahren zur Dekontaminierung zumindest einer Oberfläche eines Substrats aus festem Material, wobei die Oberfläche mit zumindest einer kontaminierenden Spezies kontaminiert ist, die sich an der Oberfläche und gegebenenfalls unter der Oberfläche (oberflächennah) in der Tiefe des Substrats befindet, wobei zumindest ein Zyklus durchgeführt wird, der die nachstehenden aufeinanderfolgenden Schritte umfasst:

    a) Auftragen des Gels nach einem der Ansprüche 1 bis 4 auf die genannte Oberfläche, beispielsweise durch Aufsprühen, Aufpinseln oder Aufstreichen;
    b) Halten des Gels an der Oberfläche zumindest für eine Zeitspanne, die ausreicht, damit das Gel die kontaminierende Spezies absorbiert und dann die kontaminierende Spezies an der Oberfläche der $TiO_2$-Teilchen adsorbiert wird und das Gel trocknet und einen trockenen und festen Rückstand bildet, der die an der Oberfläche

28

der $TiO_2$-Teilchen adsorbierte kontaminierende Spezies enthält;

c) Entfernen des trockenen und festen Rückstands, der die im Gel an der Oberfläche der $TiO_2$-Teilchen adsorbierte kontaminierende Spezies enthält, wobei der trockene und feste Rückstand beispielsweise durch Abbürsten und/oder Absaugen von der festen Oberfläche entfernt wird.

6. Verfahren nach Anspruch 5, wobei das Substrat aus zumindest einem festen Material besteht, das ausgewählt ist aus Metallen und Metalllegierungen wie rostfreiem Stahl, lackierten Stählen, Aluminium und Blei; Polymeren wie Kunststoffe oder Kautschuken wie Polyvinylchloride bzw. PVC, Polypropylene bzw. PP, Polyethylene bzw. PE, insbesondere Polyethylene hoher Dichte bzw. HDPE, Polymethylmethacrylate bzw. PMMA, Polyvinylidenfluoride bzw. PVDF, Polycarbonate bzw. PC; Glas; Zementen und zementartigen Materialien; Mörteln und Betonen; Gipsen; Ziegeln; Natur- oder Kunststein; Keramiken.

7. Verfahren nach einem der Ansprüche 5 und 6, wobei die kontaminierende Spezies ausgewählt ist aus ionischen, chemischen, biologischen, nuklearen oder radioaktiven kontaminierenden Spezies.

8. Verfahren nach Anspruch 7, wobei die kontaminierende Spezies eine ionische kontaminierende Spezies ist, ausgewählt aus ein- und mehrwertigen Metallionen, insbesondere aus toxischen ein- und mehrwertigen Metallionen, wie Chrom(VI)-, Nickel(II)-, Silber(I)-, Cadmium(II)-, Quecksilber(II)-, Arsen(III)- und Blei(II)-Ionen; ober wobei die kontaminierende Spezies eine biologische kontaminierende Spezies ist, ausgewählt aus Bakterien, Pilzen, Hefen, Viren, Toxinen, Sporen, insbesondere *Bacillus anthracis*-Sporen, Prionen und Protozoen, wobei vorzugsweise die biologische kontaminierende Spezies ausgewählt ist aus biotoxischen Spezies wie pathogenen Sporen, wie beispielsweise *Bacillus anthracis*-Sporen, Toxinen wie beispielsweise Botulinumtoxin oder Ricin, Bakterien wie *Yersinia pestis*-Bakterien und Viren wie Vaccinia-Virus oder hämorrhagische Fieberviren beispielsweise vom Ebola-Typ; oder wobei die kontaminierende Spezies ausgewählt ist aus toxischen chemischen Spezies, wie beispielsweise toxischen, insbesondere neurotoxischen oder blasenbildenden Gasen, insbesondere Organophosphorverbindungen wie Sarin oder der Wirkstoff GB, VX, Tabun oder der Wirkstoff GA, Soman, Cyclosarin, Diisopropylfluorphosphonat (DFP), Amiton oder der Wirkstoff VG, Parathion, Senfgas oder der Wirkstoff H oder der Wirkstoff HD, Lewisit oder der Wirkstoff L, der Wirkstoff T.

9. Verfahren nach einem der Ansprüche 5 bis 8, wobei das Gel auf die zu dekontaminierende Oberfläche in einer Menge von 100 g bis 2000 g Gel pro Quadratmeter Oberfläche, vorzugsweise 500 bis 1500 g Gel pro Quadratmeter Oberfläche, besonders bevorzugt 600 bis 1000 g Gel pro Quadratmeter Oberfläche, aufgetragen wird, was im allgemeinen einer Dicke des auf die Oberfläche aufgebrachten Gels von 0,1 mm bis 2 mm, vorzugsweise 0,5 mm bis 2 mm, besonders bevorzugt 1 mm bis 2 mm entspricht.

10. Verfahren nach einem der Ansprüche 5 bis 9, wobei während des gesamten oder eines Teils von Schritt a) und/oder während des gesamten oder eines Teils von Schritt b), vorzugsweise während des gesamten Schritts b), das auf der Oberfläche gehaltene Gel sichtbarer Strahlung oder ultravioletter A-, B- oder C-Strahlung (UVA, UVB oder UVC) oder anderer Strahlung ausgesetzt wird, um die kontaminierende Spezies durch Photokatalyse zu inaktivieren und/oder abzubauen und/oder zu reduzieren und/oder zu zerstören.

11. Verfahren nach einem der Ansprüche 5 bis 10, wobei (in Schritt b)) das Trocknen bei einer Temperatur von 1°C bis 50°C, vorzugsweise von 15°C bis 25°C, und unter einer relativen Feuchtigkeit von 20% bis 80%, vorzugsweise von 20% bis 70%, durchgeführt wird; wobei das Gel für einen Zeitraum von 2 bis 72 Stunden, vorzugsweise 2 bis 48 Stunden, noch bevorzugter 4 bis 24 Stunden, an der Oberfläche gehalten wird.

12. Verfahren nach einem der Ansprüche 5 bis 11, wobei der trockene, feste Rückstand in Form von Teilchen, beispielsweise Flocken, mit einer Größe von 1 bis 10 mm, vorzugsweise 2 bis 5 mm, vorliegt.

13. Verfahren nach einem der Ansprüche 5 bis 12, wobei der Zyklus 1 bis 10 Mal wiederholt wird, wobei bei allen Zyklen das gleiche Gel verwendet wird oder in einem oder mehreren Zyklen verschiedene Gele verwendet werden.

14. Verfahren nach einem der Ansprüche 5 bis 13, wobei in Schritt b) das Gel vor dem vollständigen Durchtrocknen mit einem Lösungsmittel, vorzugsweise mit dem Lösungsmittel des in Schritt a) aufgetragenen Gels, erneut befeuchtet wird.

**Claims**

1. Adsorbent and photocatalytic decontamination gel consisting of a colloidal solution comprising, preferably consisting of:

- 15 % to 20 % by weight, further preferably 15 % to 20 % by weight the 15 % value being excluded, better still 16 % to 20 % by weight, for example 20 % by weight of $TiO_2$, optionally doped, relative to the weight of the gel;
- optionally, 0.01 % to 10 % by weight, preferably 0.1 % to 5 % by weight relative to the weight of the gel of at least one dye and/or at least one pigment;
- optionally, 0.1 % to 2 % by weight relative to the weight of the gel, of at least one surfactant;
- optionally, 0.05 % to 5 % by weight, preferably 0.05 % to 2 % by weight relative to the weight of the gel, of at least one super-absorbent polymer;
- and the balance solvent, said solvent being selected from among mixtures of water in a proportion of 40% to 56% by weight, for example 40%, 42.5%, or 56% by weight, and of ethanol, in a proportion of 24% to 42,5% by weight, preferably of 24% to 40% by weight, for example of 24%, 40%, or 42.5% by weight relative to the weight of gel; and said gel having a pH of 4 or higher.

2. The gel according to claim 1 having a weak acidic pH of 4 to less than 7; or a neutral pH of 7; or a weak basic pH of more than 7 to less than 9; or a very basic pH of 9 or higher.

3. The gel according to claim 1 or 2, wherein the pH of the gel is adjusted by the addition of a mineral base, selected for example from among sodium hydroxide NaOH, potassium hydroxide KOH, and mixtures thereof.

4. The gel according to any one of the preceding claims, wherein the $TiO_2$ is in the form of particles of a mean size of 2 to 200 nm.

5. Method for decontaminating at least one surface of a substrate made of a solid material, said surface being contaminated by at least one contaminating species on said surface and optionally under said surface (in the subsurface) in the depth of the substrate, wherein at least one cycle is performed comprising the following successive steps:

a) applying the gel according to any one of claims 1 to 4 on said surface, for example by spraying, with a brush or with a float;
b) maintaining the gel on the surface at least for a sufficient time for the gel to absorb the contaminating species, for the contaminating species then to be adsorbed on the surface of the $TiO_2$ particles, and for the gel to dry and form a dry and solid residue containing said contaminating species adsorbed on the surface of the $TiO_2$ particles;
c) removing the dry and solid residue containing said contaminating species adsorbed in the gel on the surface of the $TiO_2$ particles, for example the dry and solid residue is removed from the solid surface by brushing and/or vacuuming, suction.

6. The method according to claim 5, wherein the substrate is made of at least one solid material selected from among metals and metal alloys such as stainless steel, painted steels, aluminium and lead; polymers such as plastic materials or rubbers like poly(vinyl chloride)s or PVC, polypropylenes or PP, polyethylenes or PE in particular high density polyethylenes or HDPE, poly(methyl methacrylate)s or PMMA, poly(vinylidene fluoride)s or PVDF, polycarbonates or PC; glasses; cements and cement materials; mortars and concretes; plasters; bricks; natural or artificial stone; ceramics.

7. The method according to any one of claims 5 and 6, wherein the contaminating species is selected from among ionic, chemical, biological, nuclear or radioactive contaminating species.

8. The method according to claim 7, wherein the contaminating species is an ionic contaminating species selected from among monovalent and multivalent metal ions, in particular from among toxic monovalent and multivalent metal ions such as chromium (VI), nickel (II), silver (I), cadmium (II), mercury (II), arsenic (III) and lead (II) ions; or the contaminating species is a biological contaminating species selected from among bacteria, fungi, yeasts, viruses, toxins, spores in particular spores of *Bacillus anthracis,* prions, and protozoa; preferably the biological contaminating species is selected from among biotoxic species such as pathogenic spores such as for example spores of *Bacillus anthracis,* toxins such as for example botulinum toxin or ricin, bacteria such as *Yersinia pestis* bacteria and viruses such as the vaccine virus or the viruses of haemorrhagic fevers for example of Ebola type ; or the contaminating species is selected from among toxic chemical species such as toxic gases, in particular neurotoxic or blistering, particularly organophosphorus compounds such as Sarin or agent GB, VX, Tabun or agent GA, Soman, Cyclosarin, diisopropyl fluorophosphonate (DFP), Amiton or agent VG, Parathion, mustard gas or agent H or agent HD, Lewisite or agent L, agent T.

9. The method according to any one of claims 5 to 8, wherein the gel is applied to the surface to be decontaminated in a proportion of 100 g to 2000 g of gel per $m^2$ of surface area, preferably 500 to 1500 g of gel per $m^2$ of surface area, more preferably 600 to 1000 g of gel per $m^2$ of surface area, which generally corresponds to a gel thickness deposited on the surface of 0.1 mm to 2 mm, preferably 0.5 mm to 2 mm, more preferably 1 mm to 2 mm.

10. The method according to any of claims 5 to 9, wherein during all or part of step a), and/or during all or part of step b), preferably during the entire duration of step b), the gel maintained on the surface is exposed to a visible radiation or to a A, B or C Ultraviolet radiation (UVA, UVB or UVC), or to another radiation, to inactivate, and/or degrade, and/or reduce, and/or destroy the contaminating species by photocatalysis.

11. The method according to any one of claims 5 to 10, wherein (during step b)), drying is carried out at a temperature of 1°C to 50°C, preferably 15°C to 25°C, and under a relative humidity of 20 % to 80 %, preferably 20 % to 70 %; the gel is maintained on the surface for a time of 2 to 72 hours, preferably 2 to 48 hours, more preferably 4 to 24 hours.

12. The method according to any one of claims 5 to 11, wherein the dry, and solid residue is in the form of particles, for example of flakes, of a size of 1 to 10 mm, preferably 2 to 5 mm.

13. The method according to any one of claims 5 to 12, wherein the cycle is repeated 1 to 10 times using the same gel during all cycles, or using different gels for one or more cycle(s).

14. The method according to any one of claims 5 to 13 wherein during step b) the gel, before complete drying, is rewetted with a solvent, preferably with the solvent of the gel applied during step a).

Gel TiO$_2$-H$_2$O dans le noir

FIG.1

Gel SiO$_2$-H$_2$O dans le noir

FIG.2

FIG.3

FIG.4

FIG.5

FIG.6

FIG.7

FIG.8

FIG.9

FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014154817 A1
- FR 2827530 A1
- FR 2891470 A1
- WO 2012001046 A1
- WO 2014154818 A1
- DE 102007014875 A1

**Littérature non-brevet citée dans la description**

- **L. BRANNON-PAPPAS ; R. HARLAND.** Absorbent Polymer Technology, Studies in Polymer Science. 1990, vol. 8